# EUROPEAN PATENT APPLICATION

(11) **EP 1 772 519 A1**
(43) Date of publication of application: **11.04.2007**
(21) Application number: 06077175.5
(22) Date of filing: 16.01.1998
(51) Int. Cl.: C12N 15/82, C12N 15/63

(54) **RNA binding protein and binding site useful for expression of recombinant molecules**

(30) Priority: 17.01.1997 US 35955 P; 12.12.1997 US 69400 P
(62) Divisional of application: 98903525.8
(71) Applicant: THE SCRIPPS RESEARCH INSTITUTE, La Jolla, CA 92037 (US)
(72) Inventor: Mayfield, Stephen, Cardiff, CA 92007 (US)
(74) Representative: Fisher, Adrian John

(57) **Abstract**

The present invention relates to a gene expression system in eukaryotic and prokaryotic cells, preferably plant cells and intact plants. In particular, the invention relates to an expression system having a RB47 binding site upstream of a translation initiation site for regulation of translation mediated by binding of RB47 protein, a member of the poly(A) binding protein family. Regulation is further effected by RB60, a protein disulfide isomerase. The expression system is capable of functioning in the nucleus/cytoplasm of cells and in the chloroplast of plants. Translational regulation of a desired molecule is enhanced approximately 100 fold over that obtained without RB47 binding site activation.

## Description

### Technical Field

The invention relates to expression systems and methods for expression of desired genes and gene products in cells. Particularly, the invention relates to a gene encoding a RNA binding protein useful for regulating gene expression in cells, the protein binding site, a gene encoding a regulating protein disulfide isomerase and methods and systems for gene expression of recombinant molecules.

### Background

Expression systems for expression of exogenous foreign genes in eukaryotic and prokaryotic cells are basic components of recombinant DNA technology. Despite the abundance of expression systems and their wide-spread use, they all have characteristic disadvantages. For example, while expression in *E. coli* is probably the most popular as it is easy to grow and is well understood, eukaryotic proteins expressed therein are not properly modified. Moreover, those proteins tend to precipitate into insoluble aggregates and are difficult to obtain in large amounts. Mammalian expression systems, while practical on small-scale protein production, are more difficult, time-consuming and expensive than in *E. coli.*

A number of plant expression systems exist as well as summarized in US Patent No. 5,234,834, the disclosures of which are hereby incorporated by reference. One advantage of plants or algae in an expression system is that they can be used to produce pharmacologically important proteins and enzymes on a large scale and in relatively pure form. In addition, micro-algae have several unique characteristics that make them ideal organisms for the production of proteins on a large scale. First, unlike most systems presently used to produce transgenic proteins, algae can be grown in minimal media (inorganic salts) using sunlight as the energy source. These algae can be grown in contained fermentation vessels or on large scale in monitored ponds. Ponds of up to several acres are routinely used for the production of micro-algae. Second, plants and algae have two distinct compartments, the cytoplasm and the chloroplast, in which proteins can be expressed. The cytoplasm of algae is similar to that of other eukaryotic organisms used for protein expression, like yeast and insect cell cultures. The chloroplast is unique to plants and algae and proteins expressed in this environment are likely to have properties different from those of cytoplasmically expressed proteins.

The present invention describes an expression system in which exogenous molecules are readily expressed in either prokaryotic or eukaryotic hosts and in either the cytoplasm or chloroplast. These beneficial attributes are based on the discovery and cloning of components of translation regulation in plants as described in the present invention.

Protein translation plays a key role in the regulation of gene expression across the spectrum of organisms (Kozak, Ann. Rev. Cell Biol., 8:197-225 (1992) and de Smit and Van Duin, Prog. Nucleic Acid Res. Mol. Biol., 38:1-35 (1990)). The majority of regulatory schemes characterized to date involve translational repression often involving proteins binding to mRNA to limit ribosome association (Winter et al., Proc. Natl. Acad. Sci., USA, 84:7822-7826 (1987) and Tang and Draper, Biochem., 29:4434-4439 (1990)). Translational activation has also been observed (Wulczyn and Kahmann, Cell, 65:259-269 (1991)), but few of the underlying molecular mechanisms for this type of regulation have been identified. In plants, light activates the expression of many genes. Light has been shown to activate expression of specific chloroplast encoded mRNAs by increasing translation initiation (Mayfield et al., Ann. Rev. Plant Physiol. Plant Mol Biol., 46:147-166 (1995) and Yohn et al., Mol. Cell Biol., 16:3560-3566 (1996)). Genetic evidence in higher plants and algae has shown that nuclear encoded factors are required for translational activation of specific chloroplast encoded mRNAs (Rochaix et al., Embo J., 8:1013-1021 (1989), Kuchka et al., Cell, 58:869-876 (1989), Girard-Bascou et al., Embo J., 13:3170-3181 (1994), Kim et al., Plant Mol. Biol., 127:1537-1545 (1994).

In the green algae *Chlamydomonas reinhardtii,* a number of nuclear mutants have been identified that affect translation of single specific mRNAs in the chloroplast, often acting at translation initiation (Yohn et al., supra, (1996)). Mutational analysis of chloroplast mRNAs has identified sequence elements within the 5' untranslated region (UTR) of mRNAs that are required for translational activation (Mayfield et al., supra, (1995), Mayfield et al., J. Cell Biol., 127:1537-1545 (1994) and Rochaix, Ann. Rev. Cell Biol., 8:1-28 (1992)), and the 5' UTR of a chloroplast mRNA can confer a specific translation phenotype on a reporter gene *in vivo* (Zerges and Rochaix, Mol. Cell Biol., 14:5268-5277 (1994) and Staub and Maliga, Embo J., 12:601-606 (1993).

Putative translational activator proteins were identified by purifying a complex of four proteins that binds with high affinity and specificity to the 5' UTR of the chloroplast encoded *psb*A mRNA [encoding the D1 protein, a major component of Photosystem II (PS II)] (Danon and Mayfield, Embo J., 10:3993-4001 (1991)). Binding of these proteins to the 5' UTR of *psb*A mRNA correlates with translation of this mRNA under a variety of physiological (Danon and Mayfield, id., (1991)) and biochemical conditions (Danon and Mayfield, Science, 266:1717-1719 (1994) and Danon and Mayfield, Embo J., 13:2227-2235 (1994)), and in different genetic backgrounds (Yohn et al., supra, (1996)). The binding of this complex to the *psb*A mRNA can be regulated *in vitro* in response to both redox potential (Danon and Mayfield, Science, 266:1717-1719 (1994)) and phosphorylation (Danon and Mayfield, Embo J., 13:2227-2235 (1994)), both of which are thought to transduce the light signal to activate translation of *psb*A mRNA. The 47 kDa member of the *psb*A RNA binding complex (RB47) is in close contact with the RNA, and antisera specific to this protein inhibits binding to the *psb*A mRNA *in vitro* (Danon and Mayfield, supra, (1991)).

Although the translational control of psbA mRNA by RB47 has been reported, the protein has not been extensively characterized and the gene encoding RB47 has not been identified, cloned and sequenced. In addition, the regulatory control of the activation of RNA binding activity to the binding site by nuclear-encoded trans-acting factors, such as RB60, have not been fully understood. The present invention now describes the cloning and sequencing of both RB47 and RB60. Based on the translation regulation mechanisms of RB47 and RB60 with the RB47 binding site, the present invention also describes a translation regulated expression system for use in both prokaryotes and eukaryotes.

### Brief Description of the Invention

The RB47 gene encoding the RB47 activator protein has now been cloned and sequenced, and the target binding site for RB47 on messenger RNA (mRNA) has now been identified. In addition, a regulatory protein disulfide isomerase, a 60 kilodalton protein referred to as RB60, has also been cloned, sequenced and characterized. Thus, the present invention is directed to gene expression systems in eukaryotic and prokaryotic cells based on translational regulation by RB47 protein, its binding site and the RB60 regulation of RB47 binding site activation.

More particularly, the present invention describes the use of the RB47 binding site, i.e., a 5' untranslated region (UTR) of the chloroplast *psbA* gene, in the context of an expression system for regulating the expression of genes encoding a desired recombinant molecule. Protein translation is effected by the combination of the RB47 binding site and the RB47 binding protein in the presence of protein translation components. Regulation can be further imposed with the use of the RB60 regulatory protein disulfide isomerase. Therefore, the present invention describes reagents and expression cassettes for controlling gene expression by affecting translation of a coding nucleic acid sequence in a cell expression system.

Thus, in one embodiment, the invention contemplates a RB47 binding site sequence, i.e., a mRNA sequence, typically a mRNA leader sequence, which contains the RB47 binding site. A preferred RB47 binding site is *psb*A mRNA. For use in expressing recombinant molecules, the RB47 binding site is typically inserted 5' to the coding region of the preselected molecule to be expressed. In a preferred embodiment, the RB47 binding site is inserted into the 5' untranslated region along with an upstream *psb*A promoter to drive the expression of a preselected nucleic acid encoding a desired molecule. In alternative embodiments, the RB47 binding site is inserted into the regulatory region downstream of any suitable promoter present in a eukaryotic or prokaryotic expression vector. Preferably, the RB47 binding site is positioned within 100 nucleotides of the translation initiation site. In a further aspect, 3' to the coding region is a 3' untranslated region (3' UTR) necessary for transcription termination and RNA processing.

Thus, in a preferred embodiment, the invention contemplates an expression cassette or vector that contains a transcription unit constructed for expression of a preselected nucleic acid or gene such that upon transcription, the resulting mRNA contains the RB47 binding site for regulation of the translation of the preselected gene transcript through the binding of the activating RB47 protein. The RB47 protein is provided endogenously in a recipient cell and/or is a recombinant protein expressed in that cell.

Thus, the invention also contemplates a nucleic acid molecule containing the sequence of the RB47 gene. The nucleic acid molecule is preferably in an expression vector capable of expressing the gene in a cell for use in interacting with a RB47 binding site. The invention therefore contemplates an expressed recombinant RB47 protein. In one embodiment, the RB47 binding site and RB47 encoding nucleotide sequences are provided on the same genetic element. In alternative embodiments, the RB47 binding site and RB47 encoding nucleotide sequences are provided separately.

The invention further contemplates a nucleic acid molecule containing the sequence encoding the 69 kilodalton precursor to RB47. In alternative embodiments, the RB47 nucleic acid sequence contains a sequence of nucleotides to encode a histidine tag. Thus, the invention relates to the use of recombinant RB47, precursor RB47, and histidine-modified RB47 for use in enhancing translation of a desired nucleic acid.

The invention further contemplates a nucleic acid molecule containing a nucleotide sequence of a polypeptide which regulates the binding of RB47 to RB47 binding site. A preferred regulatory molecule is the protein disulfide isomerase RB60. The RB60-encoding nucleic acid molecule is preferably in an expression vector capable of expressing the gene in a cell for use in regulating the interaction of RB47 with a RB47 binding site. Thus, the invention also contemplates an expressed recombinant RB60 protein. In one embodiment, the RB47 binding site, RB47 encoding and RB60 encoding nucleotide sequences are provided on the same genetic element. In alternative embodiments, the expression control nucleotide sequences are provided separately. In a further aspect, the RB60 gene and RB47 binding site sequence are provided on the same construct.

The invention can therefore be a cell culture system, an *in vitro* expression system or a whole tissue, preferably a plant, in which the transcription unit is present that contains the RB47 binding site and further includes a (1) transcription unit capable of expressing RB47 protein or (2) the endogenous RB47 protein itself for the purpose of enhancing translation of the preselected gene having an RB47 binding site in the mRNA. Preferred cell culture systems are eukaryotic and prokaryotic cells. Particularly preferred cell culture systems include plants and more preferably algae.

A further preferred embodiment includes (1) a separate transcription unit capable of expressing a regulatory molecule, preferably RB60 protein, or (2) the endogenous RB60 protein itself for the purpose of regulating translation of the preselected gene having an RB47 binding site in the mRNA. In an alternative preferred embodiment, one transcription unit is capable of expressing both the RB47 and RB60 proteins. In a further aspect, the RB47 binding site sequence and RB60 sequence are provided on the same construct.

In one aspect of the present invention, plant cells endogenously containing RB47 and RB60 proteins are used for the expression of recombinant molecules, such as proteins or polypeptides, through activation of the RB47 binding in an exogenously supplied expression cassette. Alternatively, stable plant cell lines containing endogenous RB47 and RB60 are first generated in which RB47 and/or RB60 proteins are overexpressed. Overexpression is obtained preferably through the stable transformation of the plant cell with one or more expression cassettes for encoding recombinant RB47 and RB60. In a further embodiment, stable cell lines, such as mammalian or bacterial cell lines, lacking endogenous RB47 and/or RB60 proteins are created that express exogenous RB47 and/or RB60.

Plants for use with the present invention can be a transgenic plant, or a plant in which the genetic elements of the invention have been introduced. Based on the property of controlled translation provided by the combined use of the RB47 protein and the RB47 binding site, translation can be regulated for any gene product, and the system can be introduced into any plant species. Similarly, the invention is useful for any prokaryotic or eukaryotic cell system.

Methods for the preparation of expression vectors is well known in the recombinant DNA arts, and for expression in plants is well known in the transgenic plant arts. These particulars are not essential to the practice of the invention, and therefore will not be considered as limiting.

The invention allows for high level of protein synthesis in plant chloroplasts and in the cytoplasm of both prokaryotic and eukaryotic cells. Because the chloroplast is such a productive plant organ, synthesis in chloroplasts is a preferred site of translation by virtue of the large amounts of protein that can be produced. This aspect provides for great advantages in agricultural production of mass quantities of a preselected protein product.

The invention further provides for the ability to screen for agonists or antagonists of the binding of RB47 to the RB47 binding site using the expression systems as described herein. Antagonists of the binding are useful in the prevention of plant propagation.

Also contemplated by the present invention is a screening assay for agonists or antagonists of RB60 in a manner analogous to that described above for RB47. Such agonists or antagonists would be useful in general to modify expression of RB60 as a way to regulate cellular processes in a redox manner.

Kits containing expression cassettes and expression systems, along with packaging materials comprising a label with instructions for use, as described in the claimed embodiments are also contemplated for use in practicing the methods of this invention.

Other uses will be apparent to one skilled in the art in light of the present disclosures.

### Brief Description of Drawings

In the figures forming a portion of this disclosure:

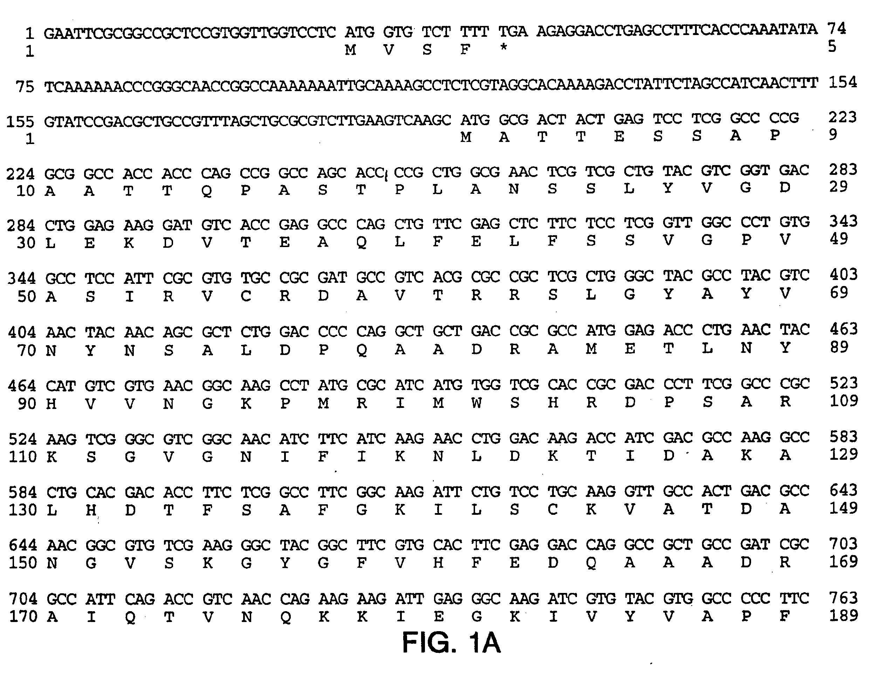

Figures 1A-1D show the complete protein amino acid residue sequence of RB47 is shown from residues 1-623, together with the corresponding nucleic acid sequence encoding the RB47 sequence, from base 1 to base 2732. The nucleotide coding region is shown from base 197-2065, the precursor form. The mature form is from nucleotide position 197-1402. Also shown is the mRNA leader, bases 1-196, and poly A tail of the mRNA, bases 2066-2732. Both the nucleotide and amino acid sequence are listed in SEQ ID NO 5.

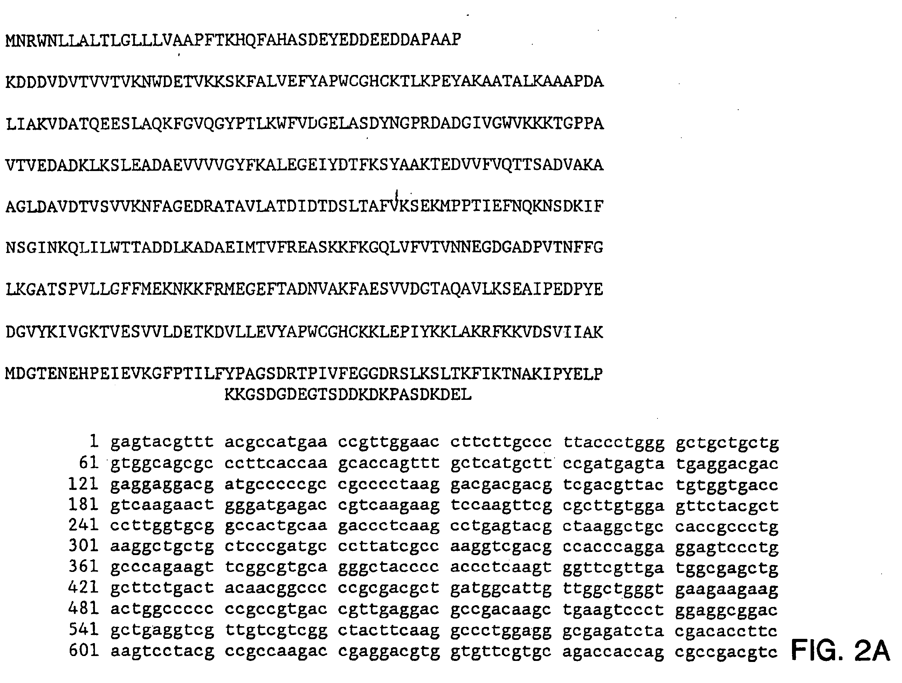

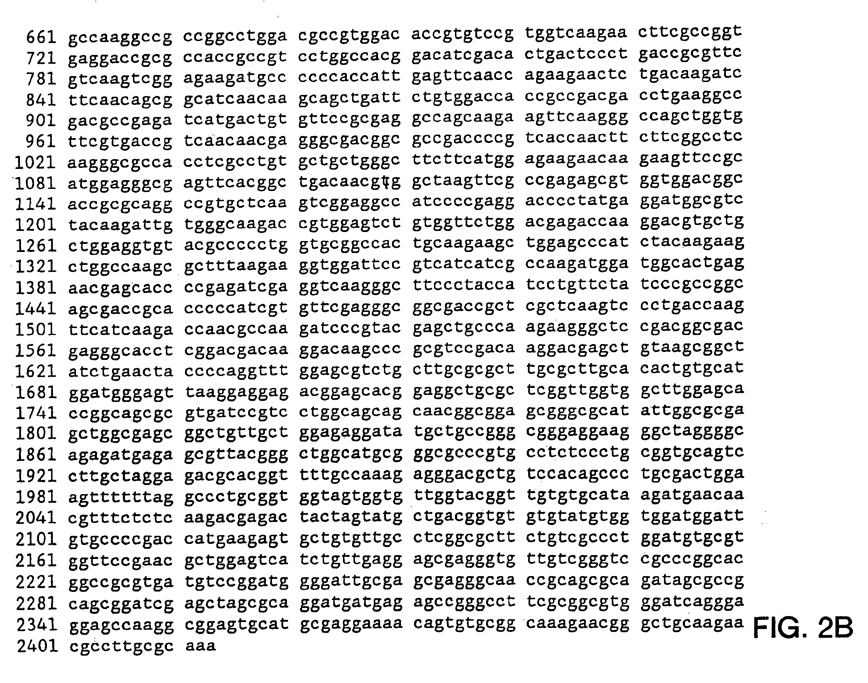

Figures 2A-2B show the complete protein amino acid residue sequence of RB60 is shown from residues 1-488, together with the corresponding nucleic acid sequence from base 1 to base 2413, of which bases 16-1614 encode the RB60 sequence. Both the nucleotide and amino acid sequence are listed in SEQ ID NO 10.

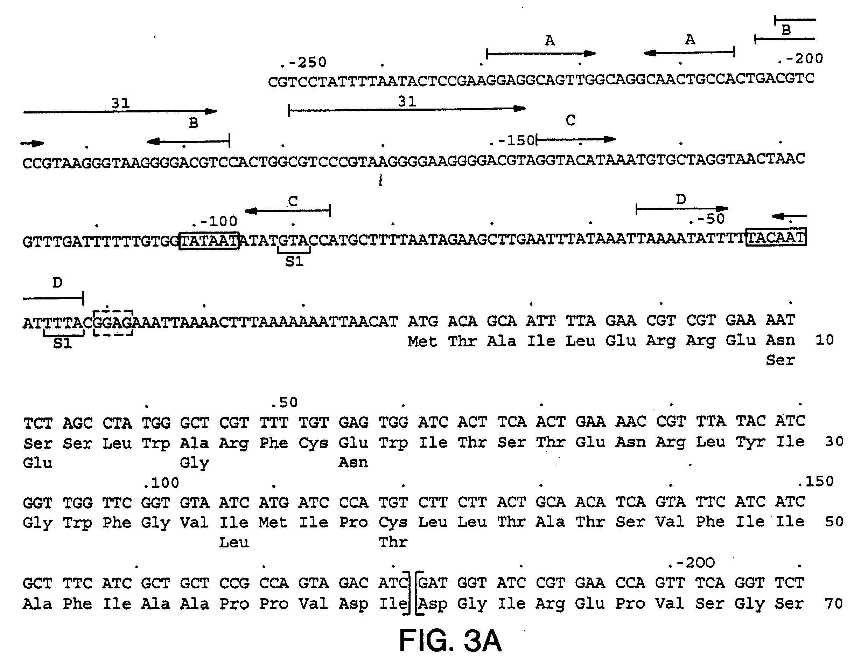

Figures 3A-3C show the complete sequence of the psbA mRNA, showing both encoded psbA protein amino acid residue sequence (residues 1-352) and the nucleic acid sequence as further described in Example 3 is illustrated. Both the nucleotide and amino acid sequence are listed in SEQ ID NO 13.

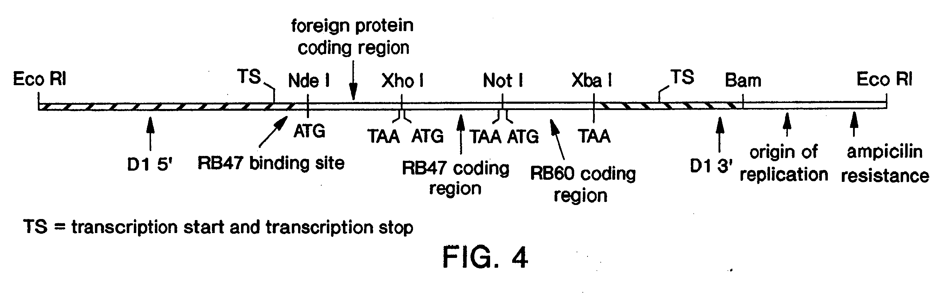

Figure 4 is a schematic diagram of an expression cassette containing on one transcription unit from 5' to 3', a promoter region derived from the *psb*A gene for encoding the D1 protein from *C. reinhardtii* further containing a transcription initiation site (TS), the RB47 binding site, a region for insertion of a foreign or heterologous coding region, a RB47 coding region, a RB60 coding region, and the 3' flanking region containing transcription termination site (TS), flanked by an origin of replication and selection marker. Restriction endonuclease sites for facilitating insertion of the independent genetic elements are indicated and further described in Example 4A.

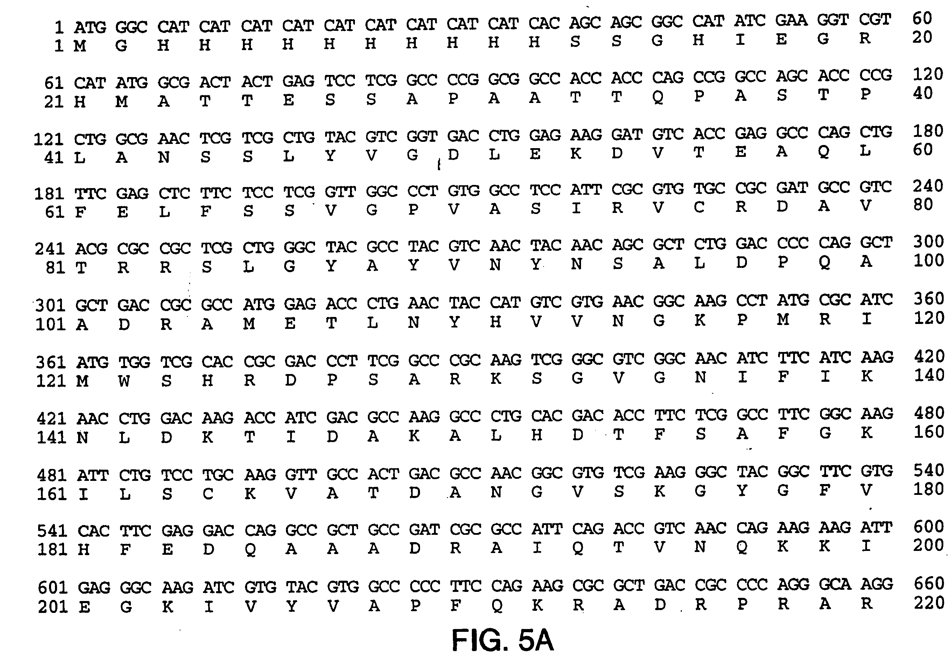

Figures 5A-5B show the nucleotide and amino acid sequence of the RB47 molecule containing a histidine tag, the sequences of which are also listed in SEQ ID NO 14.

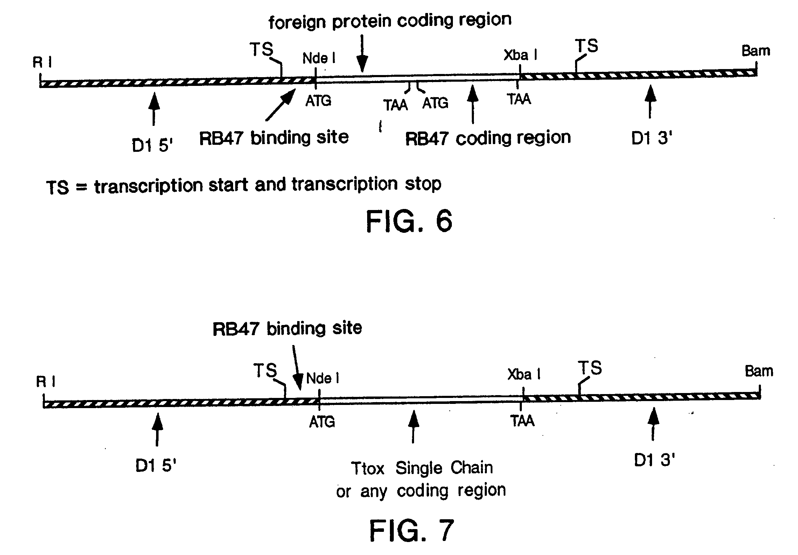

Figure 6 is a schematic diagram of an expression cassette containing on one transcription unit from 5' to 3', a promoter region derived from the *psb*A gene for encoding the D1 protein from *C. reinhardtii* further containing a transcription initiation site (TS), the RB47 binding site, a region for insertion of a foreign or heterologous coding region, a RB47 coding region, and the 3' flanking region containing transcription termination site (TS). Restriction endonuclease sites for facilitating insertion of the independent genetic elements are indicated and further described in Example 4E.
Figure 7 is a schematic diagram of an expression cassette containing on one transcription unit from 5' to 3', a promoter region derived from the *psb*A gene for encoding the D1 protein from *C. reinhardtii* further containing a transcription initiation site (TS), the RB47 binding site, a region for insertion of a foreign or heterologous coding region, and the 3' flanking region containing transcription termination site (TS). Restriction endonuclease sites for facilitating insertion of the independent genetic elements are indicated and further described in Example 4G.

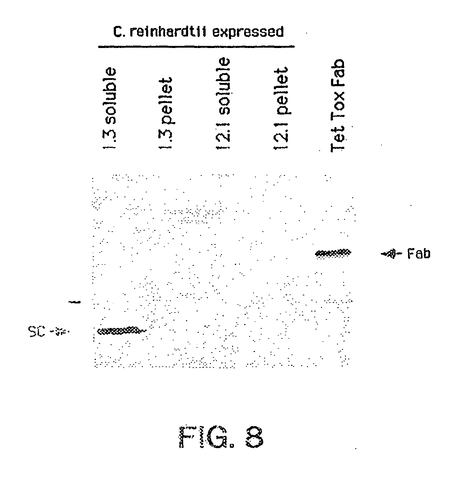

Figure 8 is a Western blot of a tetanus toxin single chain antibody expressed with a construct of the present invention as further described in Example 4G1).

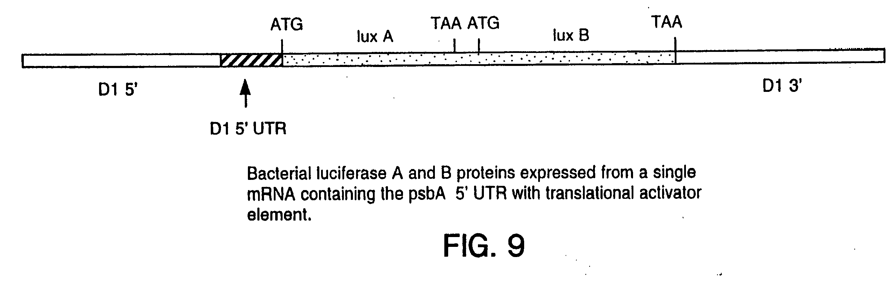

Figure 9 is a schematic diagram of an expression cassette containing on one transcription unit from 5' to 3', a promoter region derived from the *psb*A gene for encoding the D1 protein from *C. reinhardtii* further containing a transcription initiation site (TS), the RB47 binding site, a region for insertion of a coding sequence of bacterial luciferase A and B proteins including the translation termination codon TAA. The 3' flanking region containing transcription termination site (TS). Restriction endonuclease sites for facilitating insertion of the independent genetic elements are indicated and further described in Example 4G2).

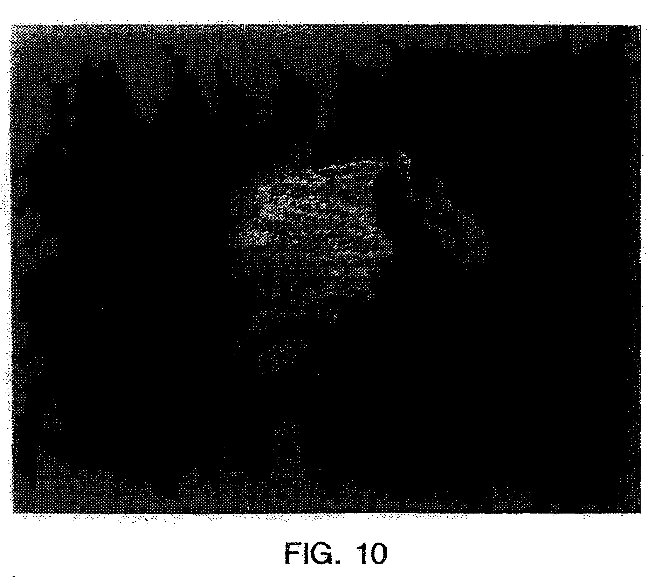

Figure 10 illustrates the accumulation of expressed bacterial luciferase protein in the chloroplast as further described in Example 4G2).

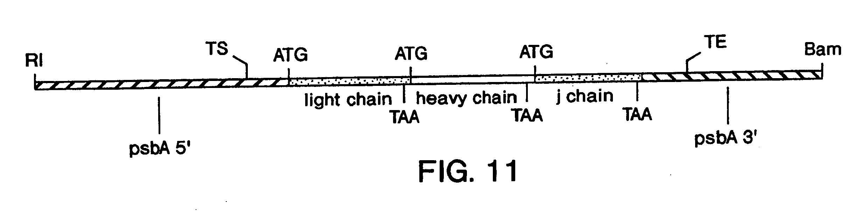

Figure 11 is a schematic diagram of an expression cassette containing on one transcription unit from 5' to 3', a promoter region derived from the *psb*A gene for encoding the D1 protein from *C. reinhardtii* further containing a transcription initiation site (TS), the RB47 binding site, a region for insertion of a foreign or heterologous coding region for dimeric IgA (dIgA) and the 3' flanking region containing transcription termination site (TS). Restriction endonuclease sites for facilitating insertion of the independent genetic elements are indicated and further described in Example 4G3).

### Detailed Description of the Invention

### A. Definitions

**TABLE OF CORRESPONDENCE**

| Code | Group | Nucleotide(s) |
|---|---|---|
| A | A | adenine |
| C | C | cytosine |
| G | G | guanine |
| T | T | thymine (in DNA) |
| U | U | uracil (in RNA) |
| Y | C or T(U) | pyrimidine |
| R | A or G | purine |
| M | A or C | amino |
| K | G or T(U) | keto |
| S | G or C | strong interaction (3 hydrogen bonds) |
| W | A or T(U) | weak interaction (2 hydrogen bonds) |
| H | A or C or T(U) | not-G |
| B | G or T(U) or C | not-A |
| V | G or C or A | not-T or not-U |
| D | G or A or T(U) | not-C |
| N | G, A, C or T(U) | any |

Amino Acid Residue: An amino acid formed upon chemical digestion (hydrolysis) of a polypeptide at its peptide linkages. The amino acid residues described herein are preferably in the "L" isomeric form. However, residues in the "D" isomeric form can be substituted for any L-amino acid residue, as long as the desired functional property is retained by the polypeptide. NH₂ refers to the free amino group present at the amino terminus of a polypeptide. COOH refers to the free carboxy group present at the carboxy terminus of a polypeptide. In keeping with standard polypeptide nomenclature (described in J. Biol. Chem., 243:3552-59 (1969) and adopted at 37-CFR §1.822 (b) (2)), abbreviations for amino acid residues are shown in the following Table of Correspondence:

**TABLE OF CORRESPONDENCE**

| SYMBOL | | AMINO ACID |
|---|---|---|
| 1-Letter | 3-Letter | |
| Y | Tyr | tyrosine |
| G | Gly | glycine |
| F | Phe | phenylalanine |
| M | Met | methionine |
| A | Ala | alanine |
| S | Ser | serine |
| I | Ile | isoleucine |
| L | Leu | leucine |
| T | Thr | threonine |
| V | Val | valine |
| P | Pro | proline |
| K | Lys | lysine |
| H | His | histidine |
| Q | Gln | glutamine |
| E | Glu | glutamic acid |
| Z | Glx | Glu and/or Gln |
| W | Trp | tryptophan |
| R | Arg | arginine |
| D | Asp | aspartic acid |
| N | Asn | asparagine |
| B | Asx | Asn and/or Asp |
| C | Cys | cysteine |
| X | Xaa | unknown/other |

In addition the following have the meanings below:
- BOC: tert-butyloxycarbonyl
- DCCI: dicylcohexylcarbodiimide
- DMF: dimethylformamide
- OMe: methoxy
- HOBt: 1-hydroxybezotriazole

It should be noted that all amino acid residue sequences are represented herein by formulae whose left and right orientation is in the conventional direction of amino-terminus to carboxy-terminus. Furthermore, it should be noted that a dash at the beginning or end of an amino acid residue sequence indicates a peptide bond to a further sequence of one or more amino acid residues.

Polypeptide: A linear series of amino acid residues connected to one another by peptide bonds between the alpha-amino group and carboxy group of contiguous amino acid residues.

Peptide: A linear series of no more than about 50 amino acid residues connected one to the other as in a polypeptide.

Protein: A linear series of greater than 50 amino acid residues connected one to the other as in a polypeptide.

Synthetic peptide: A chemically produced chain of amino acid residues linked together by peptide bonds that is free of naturally occurring proteins and fragments thereof.

Nucleotide: A monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. When the nucleoside contains a phosphate group bonded to the 3' or 5' position of the pentose it is referred to as a nucleotide. A sequence of operatively linked nucleotides is typically referred to herein as a "base sequence" or "nucleotide sequence", and their grammatical equivalents, and is represented herein by a sequence whose left to right orientation is in the conventional direction of 5'-terminus to 3'-terminus.

Base Pair (bp): A partnership of adenine (A) with thymine (T), or of cytosine (C) with guanine (G) in a double stranded DNA molecule. In RNA, uracil (U) is substituted for thymine.

Nucleic Acid: A polymer of nucleotides, either single or double stranded.

Polynucleotide: A polymer of single or double stranded nucleotides. As used herein "polynucleotide" and its grammatical equivalents will include the full range of nucleic acids. A polynucleotide will typically refer to a nucleic acid molecule comprised of a linear strand of two or more deoxyribonucleotides and/or ribonucleotides. The exact size will depend on many factors, which in turn depends on the ultimate conditions of use, as is well known in the art. The polynucleotides of the present invention include primers, probes, RNA/DNA segments, oligonucleotides or "oligos" (relatively short polynucleotides), genes, vectors, plasmids, and the like.

Gene: A nucleic acid whose nucleotide sequence codes for an RNA or polypeptide. A gene can be either RNA or DNA.

Duplex DNA: A double-stranded nucleic acid molecule comprising two strands of substantially complementary polynucleotides held together by one or more hydrogen bonds between each of the complementary bases present in a base pair of the duplex. Because the nucleotides that form a base pair can be either a ribonucleotide base or a deoxyribonucleotide base, the phrase "duplex DNA" refers to either a DNA-DNA duplex comprising two DNA strands (ds DNA), or an RNA-DNA duplex comprising one DNA and one RNA strand.

Complementary Bases: Nucleotides that normally pair up when DNA or RNA adopts a double stranded configuration.

Complementary Nucleotide Sequence: A sequence of nucleotides in a single-stranded molecule of DNA or RNA that is sufficiently complementary to that on another single strand to specifically hybridize to it with consequent hydrogen bonding.

Recombinant DNA (rDNA) molecule: A DNA molecule produced by operatively linking two DNA segments. Thus, a recombinant DNA molecule is a hybrid DNA molecule comprising at least two nucleotide sequences not normally found together in nature. rDNA's not having a common biological origin, i.e., evolutionarily different, are said to be "heterologous".

Vector: A rDNA molecule capable of autonomous replication in a cell and to which a DNA segment, e.g., gene or polynucleotide, can be operatively linked so as to bring about replication of the attached segment. Vectors capable of directing the expression of genes encoding for one or more polypeptides are referred to herein as "expression vectors".

Conserved: A nucleotide sequence is conserved with respect to a preselected (reference) sequence if it non-randomly hybridizes to an exact complement of the preselected sequence.

Hybridization: The pairing of substantially complementary nucleotide sequences (strands of nucleic acid) to form a duplex or heteroduplex by the establishment of hydrogen bonds between complementary base pairs. It is a specific, i.e., non-random, interaction between two complementary polynucleotides that can be competitively inhibited.

Nucleotide Analog: A purine or pyrimidine nucleotide that differs structurally from A, T, G, C, or U, but is sufficiently similar to substitute for the normal nucleotide in a nucleic acid molecule.

Upstream: In the direction opposite to the direction of DNA transcription, and therefore going from 5' to 3' on the noncoding strand, or 3' to 5' on the RNA transcript.

Downstream: Further along a DNA sequence in the direction of sequence transcription or read out, that is, traveling in a 3'- to 5'-direction along the noncoding strand of the DNA or 5'- to 3'-direction along the RNA transcript.

Stop Codon: Any of three codons that do not code for an amino acid, but instead cause termination of protein synthesis. They are UAG, UAA and UGA and are also referred to as a nonsense, termination, or translational stop codon.

Reading Frame: Particular sequence of contiguous nucleotide triplets (codons) employed in translation. The reading frame depends on the location of the translation initiation codon.

Homolog: Refers to a molecules that is structurally or functionally equivalent to a molecule of the present invention.

Fusion Protein: A polypeptide produced by recombinant DNA methods in which a first polypeptide domain is operatively linked to a second polypeptide domain by the peptide bond produced through expression of a single open reading frame to express a single "fused" polypeptide.

Chimeric Molecule: A bifunctional molecule formed by connecting two separate molecules through chemical linkage, such as by crosslinking two isolated polypeptides or joining two heterologous fragments of DNA from different sources.

### B. Translational Regulation by RB47 and RB60

### 1. RB47 and RB47 Binding Site

The present invention is based primarily on the discovery of that RB47, a mRNA binding protein, is a translational activator of the chloroplast *psb*A mRNA from *Chlamydomonas reinhardtii,* a green algae. The role of RB47 is now clearer, as evidence for its function comes from several independent sources. First, biochemical analysis has shown that this protein (along with other proteins within the complex) binds with high affinity and specificity to the *psb*A 5' UTR in a manner consistent with a role in translational regulation; high levels of binding are observed during high translation levels in the light, and low levels of binding are observed when translation is low in the dark. Second, the predicted amino acid sequence of RB47, now available since the cloning of RB47 as described herein, indicates the role this protein plays in translation. RB47 belongs to a family of proteins known as poly(A) binding proteins that bind RNA and have been shown to play a role in translation initiation (Bag and Wu, Eur. J. Biochem., 237:143-152 (1996); de Melo Neto et al., Nuc. Acids Res., 23:2198-2205 (1995); Proweller and Butler, J. Biol. Chem., 271:10859-10865 (1996); Sachs and Davis, Cell, 58:857-867 (1989); Tarun and Sachs, Embo J., 15:7168-7177 (1996)). Finally, genetic analysis has predicted translational activators of chloroplast mRNAs (Girard-Bascou et al., Curr. Genet., 22:47-52 (1992); Kuchka et al., Embo J., 7:319-324 (1988); Rochaix et al., Ann. Rev. Cell Biol., 8:1-28 (1989); and Yohn et al., Mol. Cell. Biol., 16:3560-3566 (1996)). With the use and characterization of the hf149 mutant, a high fluorescence phenotype of *C. reinhardtii;* the absence of RB47 has been shown to correspond directly to the loss of translational initiation of the *psbA* mRNA, thus defining RB47 as a translational activator of the *psb*A mRNA. This is further supported by an additional nuclear mutation in *C. reinhardtii* (hf261) which is non-allelic to hf149, but shows the similar phenotype of a specific loss of D1 translation. The RB47 protein accumulates to less than 10% of the wild type level in this mutant.

While proteins which bind to the 5' UTR of chloroplast mRNAs seem likely candidates for translational activators, no direct link had been made to the body of genetic data prior to the characterization of the hf149 mutant as described in the present invention. Thus, the identification of RB47, the cloning thereof, and the role of RB47 in translation activation of *psbA* are novel and form the basis of the mechanisms of the expression cassettes of the present invention.

hf149 is not likely to be a mutation directly in the RB47 gene, as Southern and Northern analysis indicates that the RB47 gene is intact and produces normal amounts of RB47 mRNA in the hf149 strain. This leaves open the possibility that the loss of RB47 protein is the result of a loss of *psbA* translation, rather than the cause of it. Although this is a formal possibility, it is highly unlikely given the fact that the RB47 protein accumulates in other *psb*A translation initiation deficient mutants (e.g. F35, Yohn et al., Mol. Cell Biol., 16:3560-1566 (1996)), and that the *psbA* RB60 RNA binding protein still accumulates in the hf149 strain. Thus, the hf149 mutation provides strong evidence that the RB47 protein is directly involved in translational regulation of the chloroplast encoded *psbA* mRNA. Identification of the specific defect in the hf149 mutant should yield further insights into this process.

The dramatic reduction in the amount of *psb*A mRNA associated with ribosomes in the hf149 mutation suggests that RB47 is specifically required for ribosomes to initiate translation with the *psbA* mRNA. Although the identification of a message specific translational activator in the chloroplast has not previously been shown, other organellar systems may use similar mechanisms for controlling and coordinating gene expression, most notably the mitochondria of yeast. In particular, the *COX3* mRNA of *Saccharomyces cerevisiae* is translationally regulated by a complex of at least three proteins which have been shown genetically (Wiesenberger et al., Mol. Cell Biol., 15:3291-3300 (1995)) and biochemically (Brown et al., Mol. Cell Biol.,14:1045-1053 (1994)) to interact with each other and with the *COX3* mRNA. One of these proteins (PET122) also interacts with the mitochondrial ribosome (Haffter et al., Genetics, 127:319-326 (1991); Haffter et al., Genetics, 125:495-503 (1990); McMullin et al., Mol. Cell Biol., 10:4590-4595 (1990)), suggesting a model for translational activation in which these proteins facilitate the initial interaction between the mRNA and the ribosome. A similar mechanism may be involved with RB47, the *psb*A mRNA and chloroplast ribosomes.

The identification of RB47 as a poly(A) binding protein (PABP) is somewhat unexpected given that translation in the chloroplast is generally considered prokaryotic like, and PABPs have not been identified as components of the prokaryotic translation apparatus. The chloroplast has 70S ribosomes (as in prokaryotes) and the mRNAs encoded by the chloroplast genome do not, in general, have poly(A) tails, and often contain prokaryotic consensus ribosome binding sequences (Gillham et al., Ann. Rev. Genetics, 28:71-93 (1994); Harris et al., Microbiol. Rev., 58:700-754 (1994)). The addition of A-rich sequences to the 3' end of endonucleolytic cleavage products of some chloroplast mRNAs has recently been described (Kudla et al., Embo J., 15:7137-7146 (1996); Lisitsky et al., Proc. Natl. Acad. Sci., USA, 93:13398-13403 (1996)), and this seems to play a role in degradation of the RNA, as in prokaryotes. The identification of a PABP in the chloroplast indicates that components of the cytoplasmic translation machinery may have been appropriated by the chloroplast for a similar function. These data also indicate that PABPs may function in translational regulation in the chloroplast in a manner not previously described for cytoplasmic mRNAs, although the role of RB47 in *psb*A translation seems to fit with the limited information known about the function of PABPs in other systems. While no specific biochemical function has yet been identified for any member of the PABP family, these proteins have been defined as specific RNA binding proteins with a role in translational regulation. In yeast, PABP is essential for viability (Sachs et al., Cell, 45:827-835 (1986); Sachs et al., Mol. Cell Biol., 7:3268-3276 (1987)), and a temperature sensitive (ts) allele of PABP shows that depletion of PABP in yeast results in inhibition of translation initiation and poly(A) tail shortening (Sachs and Davis, Cell, 58:857-867 (1989)). Further, revertants of this ts mutation mapped to a ribosomal protein, suggesting that PABP interacts with the ribosome to mediate translation initiation (Sachs and Davis, id., (1989)). In addition, PABPs have been shown to physically interact with ribosomes (Proweller and Butler, supra, (1996)), and with eukaryotic initiation factors (eIF4G) (Tarun and Sachs, supra, (1996)). RB47 appears to fit these general roles predicted for PABPs, with the exception that RB47 shows specific binding to the 5' UTR of the *psb*A mRNA, and that RB47 is acting in the chloroplast, where translation is distinct from that in the cytoplasm both spatially and mechanistically. However, the fact that this nuclear encoded, eukaryotic protein has been exploited for use in the chloroplast may not be too surprising given the bi-directional exchange of genetic information between the chloroplast and nucleus (Morden et al., Biosystems, 28:75-90 (1992)).

Thus, in view of the binding specificity of RB47 to the RB47 binding site in *psb*A mRNA, the present invention is unique in describing expression cassettes regulated at the translational level.

From the genetic, biochemical and molecular analysis of translational regulation in the chloroplast, a model for how the *psb*A mRNA binding proteins act in translation initiation and activation of *psb*A mRNA is presently formulated. In this model, nuclear encoded proteins, including the PABP homologue RB47, are transported into the chloroplast. Once in the plastid these proteins are activated to bind to RNA elements found within the 5' UTR of specific mRNAs (Mayfield et al., J. Cell Biol., 127:1537-1545 (1994)). This activation of binding is light responsive via the reducing potential generated by the light reactions of photosynthesis (Danon and Mayfield, Embo J., 13:2227-2235 (1994)). The interaction of the translational activator proteins and cis-acting RNA elements facilitates the initial interaction of the message with ribosomal subunits, resulting in increased translation of the D1 protein from the *psb*A mRNA.

Thus, in view of the enhancement of translation by the binding of a translation activator protein on a RNA element and in view of the cloning of both RB47 and RB60 translation activator proteins, the present invention contemplates the following aspects related to expression systems and uses thereof: isolated nucleic acids encoding recombinant proteins and variations thereof; the recombinant proteins themselves; use of the RNA binding site element in concert with the RB47 and RB60 nucleic acids and proteins thereof including endogenously expressed counterparts; expression cassettes in which the genetic elements of this invention are operably linked; expression systems including cells *in vitro* and *in vivo*; methods of use thereof for expressing a heterologous molecule and for screening for agonists and antagonists of the interaction on which the present invention is based; and lastly, kits for use in expression of proteins and preparation of RNA transcripts.

The present invention therefore describes the use of an RB47 binding site nucleotide sequence and a coordinate RB47 binding site molecule for the purpose of enhancing translation of a desired heterologous coding sequence, thereby producing the desired expressed molecule for use thereafter.

Based on the translational activation mediated through the binding of an RB47 binding site sequence, typically a mRNA sequence, the present elements of RB47 binding site and an RB47 binding site polypeptide are therefore referred to as a translational activation system. The system is capable of further modulation or regulation by a polypeptide that regulates the binding of a RB47 binding site interaction with its activator protein as discussed in the next section. In a preferred embodiments, the translational activator protein of RB47 binding site is RB47 and the translation regulatory molecule that regulates the binding of an activator to an RB47 binding site is RB60, the latter of which is discussed below.

Thus, a molecule that binds RB47 and results in the translational activation of RB47 binding site thereby enhancing translation of a desired mRNA sequence is referred to as a RB47 binding site polypeptide. Preferably, the polypeptide is RB47 that is present endogenously, i.e., naturally occurring, in a cell such that activation of a RB47 binding site occurs through an interaction of an endogenous protein with an exogenously provided RB47 binding site sequence as further described below. Functional RB47 protein is found in plant chloroplasts as reviewed above.

In other embodiments, RB47 is a recombinant protein produced through the expression of the coding sequence in a recipient cell as discussed in Section C below. Expression of a recombinant RB47 is now possible in view of the cloning of the cDNA encoding of RB47 as described in the present invention and in Examples 2 and 3. Exemplary recombinant RB47 proteins produced by the methods of this invention, more completely described in the Examples, include mature or processed RB47 that is approximately a 47 kilodalton (kDa) protein, precursor or unprocessed RB47 that is approximately a 69 kDa protein, and a histidine-modified RB47 protein that is also approximately a 47 kDa protein, the latter of which is useful for purification aspects as described in the Examples.

Thus, although the preferred RB47 proteins and nucleic acid compositions are derived from *Chlamydomonas reinhardtii* as discussed in the present invention, variations at both the amino acid and nucleotide sequence level may exist in similar functioning molecules isolated from different algae species as well as within differing plant geni. Such variations are not to be construed as limiting. For example, allelic variation within a plant species can tolerate a several percent difference between isolates of a type of RB47, the differences of which comprise non-deleterious variant amino acid residues. Thus a protein of about 95% homology, and preferably at least 98% homology, to a disclosed RB47 protein is considered to be an allelic variant of the disclosed RB47 protein, and therefore is considered to be a RB47 protein of this invention.

Thus, the term "homolog" refers to any RB47-like protein or polypeptide having similar three-dimensional structure based on the amino acid residue sequence that can be encoded by differing specific nucleic acid sequences. In other words, the RB47 species of this invention are homologous molecules in view of the amino acid sequence similarity, the presence of a species specific sequence, the overall secondary and tertiary structure of the molecule, and the like physical parameters.

Thus as used herein, the phrases "RB47 protein" and "RB47 peptide or polypeptide" refers to a RB47 molecule having an amino acid residue sequence that comprises an amino acid residue sequence that corresponds, and preferably is identical, to a portion of a RB47 protein, either produced endogenously or exogenously to produce recombinant proteins, of this invention.

A recombinant RB47 protein need not necessarily be substantially pure, or even isolated, to be useful in certain embodiments, although recombinant production methods are a preferred means to produce a source for further purification to yield an isolated or substantially pure receptor composition. A recombinant RB47 protein can be present in or on a mammalian cell line or in crude extracts of a mammalian cell line. In other embodiments, a recombinant RB47 protein is produced in or on plants or plant cell lines, for subsequent use therein to activate the translation of a desired coding sequence as described in Section C. Preferred expression vector systems for production of RB47 proteins of this invention in this context are described in Section C and in the Examples.

In the context of the present translational activation system of this invention, the presence of a RB47 binding site sequence is required. Thus, a RB47 binding site sequence is referred to as a translational activation binding domain, the activation of which leads to the enhanced or increased translation of a desired coding sequence. As previously discussed, endogenous activation of the RB47 mRNA binding sequence in the *psb*A gene in green algae chloroplast by RB47 results in the expression of D1 protein. As further discussed below, this translation activation can be further modulated or regulated by RB60. The nucleotide sequence in the 5' untranslated (5' UTR) end of the *psb*A gene containing the RB47 binding site is described in Example 3. The use of the RB47 binding site sequence thus is contemplated for use in preparing an expression cassette of this invention as further described in Section C and more completely in the Examples. Insofar as the binding of a RB47 binding site polypeptide to the nucleotide sequence of the RB47 binding site allows for translational activation of an encoding mRNA, variations, substitutions, additions, deletions and the like permutations in the nucleic acid sequence of the RB47 nucleic acid sequence are contemplated for use in the present invention. In addition, any functional RB47 binding site nucleotide sequence is generally positioned upstream, i.e., 5', to the desired coding nucleotide sequence and in relation to the other inserted genetic elements including an upstream promoter, transcription initiation sites and downstream translation initiation sites of a coding region that can be a desired coding sequence or one of the genetic control elements of the invention, such as RB47 or RB60, as further described in Section C. and in the Examples.

### 2. RB60

Light-regulated translation of chloroplast mRNAs requires trans-acting factors that interact with the 5' untranslated region (UTR) of these mRNAs. The present invention describes a protein disulfide isomerase (PDI), also referred to as RB60, that is localized to the chloroplast and co-purifies with cPABP. The cDNA encoding the RB60 protein has now been cloned as described herein. As described more fully below, the RB60 protein has now been shown to modulate the binding of RB47, the cPABP, to the 5' UTR of the *psb*A mRNA by reversibly changing the redox status of cPABP using redox potential or ADP-dependent phosphorylation. This mechanism allows for a simple reversible switch regulating gene expression in the chloroplast. Moreover, in view of the modulatory properties of RB60 as discussed below, incorporation of RB60 into the compositions and methods of this invention are valuable for regulating the expression of a desired gene product with the expression cassettes and systems of the present invention as described further herein and in the Examples.

The present inventors have determined the role of RB60 in regulating the binding of RB47 to *psb*A mRNA containing the RB47 binding site. The work has recently been published, Kim and Mayfield, Science, 278:1954-1957 (1997), references for the RB60 section herein are provided in the published paper. As previously discussed, synthesis of certain chloroplast photosynthetic proteins is activated 50-100 fold in response to light exposure without an increase in the corresponding mRNA levels, indicating that translation of chloroplast mRNAs is light-regulated. Genetic evidence has shown that nuclear-encoded trans-acting factors interact with the 5' untranslated region (UTR) of chloroplast mRNAs to activate translation of these mRNAs in a light-dependent fashion. A set of proteins (38, 47, 55 and 60 kDa) was identified to bind as a complex to the 5' UTR of the *psb*A mRNA, encoding the photosynthetic reaction center protein D1 from the green algae *Chlamydomonas reinhardtii*. Binding of this protein complex to the 5' UTR of the *psb*A mRNA correlates with light-enhanced translation of this mRNA under a variety of environmental condition, and in mutations deficient in *psb*A mRNA translation. RNA binding activity of the protein complex for the 5' UTR of the *psb*A mRNA can be regulated *in vitro* by at least two different mechanisms: ADP-dependent phosphorylation and changes in redox potential.

The present invention and the Kim and Mayfield, id., (1997) paper describe the cloning of the cDNA encoding the 60 kDa *psb*A mRNA binding protein (RB60) as further described in the Examples. The predicted amino acid sequence of the cloned cDNA is also described therein.

To verify that RB60 is localized to the chloroplasts, an immunoblot analysis of isolated pea chloroplasts was performed using the *C. reinhardtii* RB60 antiserum. To confirm that the isolated pea chloroplasts were free of cytoplasmic contamination, immunoblot analysis was performed with antiserum against the large subunit of ribulose bisphosphate carboxylase (RuBPCase, located in chloroplast) and antiserum against the cytoplasmic protein tubuli. RuBPCase antiserum recognized proteins from both whole leaf extracts (cytoplasm plus chloroplast) and from isolated chloroplasts. The tubulin antiserum recognized a protein in whole leaf extracts, but not in the chloroplast fraction), showing that the isolated chloroplasts were free of cytoplasmic proteins. The protein extracts from isolated pea chloroplasts were enriched using heparin-agarose chromatography: enrichment was required for immunoblot assays with the RB60 antiserum as RB60 is a minor component within the chloroplast. Immunoblot analysis was performed on proteins from purified pea chloroplasts, from *C. reinhardtii* cell extracts isolated by heparin-agarose chromatography, and on recombinant RB60. A specific signal immunochemically related to RB60 was clearly detected at approximately 63 kDa in the pea chloroplast sample. A signal of equal intensity was observed for *C. reinhardtii* proteins and for the recombinant RB60.

Chloroplast PDI (cPDI) contains the two -CGHC-catalytic sites that are involved in the formation, reduction and isomerization of disulfide bonds associated with protein folding. The identification of these redox catalytic sites prompted the'investigation of the role of RB60 in the redox-regulated binding of RB47 to the 5' UTR of the *psb*A mRNA. Both RB60 and RB47, containing only the four RNA recognition motif domains, were expressed as further described in the Examples in *E. coli* as a fusion protein with a histidine tag, purified on a Ni-NTA agarose affinity column and used for subsequent RNA binding gel mobility-shift assays. The effect of a reducing agent on RNA binding activity of recombinant RB47 (r-RB47) was assessed by the addition-of DTT (dithiothreitol) in the presence of recombinant RB60 (r-RB60). r-RB47 was preincubated with 10 mM DTT, a 5-fold excess of r-RB60 alone, or both DTT plus r-RB60, prior to adding a ³²P-labeled 5'-UTR of the *psb*A mRNA, followed by a gel mobility-shift assay. The results showed that r-RB47 isolated from *E. coli* was in an active reduced form so that only a slight enhancement of RNA binding activity was obtained with addition of DTT and r-RB60.

To determine whether r-RB60 was able to re-activate r-RB47 that was in an inactive oxidized form, r-RB47 was incubated with the oxidant dithionitrobenzoic acid (DTNB) for 5 minutes and then dialyzed against 10⁴ volume of buffer to remove the oxidant. Oxidation of r-RB47 by DTNB completely abolished the binding activity of the protein. Addition of DTT to 1.0 mM partially restored the binding capacity of r-RB47, and the binding was increased three fold by the addition of up to 25 mM DTT. With increasing amounts of r-RB60, the binding activity of r-RB47 was increased compared to the samples without r-RB60 at every level of DTT tested. When DTT was not present in the incubation medium, r-RB60 alone could not restore the binding of the oxidized r-RB47 (0 mM DTT), indicating that r-RB60 requires reducing equivalents to convert the inactive oxidized form of r-RB47 to an active reduced form.

Protein disulfide isomerase is known to catalyze the formation of disulfide bonds by oxidation of the sulfhydryl groups of cysteine residues during protein folding. To examine whether r-RB60 was also capable of oxidative catalysis of the reduced form of r-RB47, GSSG, the oxidized form of the thiol tripeptide glutathionine, was added to the assay mixture. When GSSG alone was added to r-RB47 at up to 5 mM, there was a two fold reduction in binding activity of r-RB47 compared with untreated protein. Incubation of r-RB47 with both GSSG and r-RB60 reduced the binding activity of r-RB47 by 5-6 fold, indicating that r-RB60 can facilitate the conversion of the reduced form of r-RB47 to an inactive oxidized form under an oxidizing environment. Thus, RB60 modulates or in other words regulates the redox potential essential for RB47 binding activity. As such, RB60 is a regulatory protein useful in regulating the expression of a desired coding sequence in reducing and oxidizing environments as supported by the teachings described herein.

ADP-dependent phosphorylation of RB60 has previously been shown to reduce binding of the protein complex to the 5'-UTR of the *psb*A mRNA. To identify if recombinant RB60 can be phosphorylated, r-RB60 was incubated with heparin-purified proteins from C. *reinhardtii* in the presence of g-³²P-ATP. Phosphorylated r-RB60 was detected among a number of phosphorylated proteins in the heparin-purified fraction. Purification of the incubation mixtures on Ni-NTA resin resulted in the isolation of phosphorylated r-RB60. Phosphorylated r-RB60 was able to reduce the binding of r-RB47 to the 5' UTR of the *psb*A mRNA, whereas, phosphorylated *C. reinhardtii* proteins eluted from Ni-NTA resin had little impact on r-RB47 RNA binding.

It has previously been shown that thioredoxin can act as a transducer of redox potential to enhance the binding of a protein complex to the *psb*A mRNA. PDI fits well into this scheme as ferredoxin-thioredoxin reductase is capable of directly reducing PDI.

In a functional model of RB60 regulation, reducing equivalents, generated by photosynthesis, are donated to cPDI (RB60) through ferredoxin and ferredoxin-thioredoxin reductase and act to catalyze the reduction of chloroplast poly(A) binding protein (cPABP) (RB47). The reduced form of cPABP is then capable of binding to the 5' UTR of the *psb*A mRNA to activate translation initiation of this mRNA resulting in increased synthesis of the D1 protein. This mechanism provides a direct link in the chloroplast between the quantity of absorbed light and the rate of synthesis of the D1 protein, allowing the replacement of the photo-damaged D1 protein. Protein disulfide isomerase has an additional advantage in this scheme in that it has greater oxidation potential than thioredoxin, thus allowing the off switch (oxidation) when reducing potential is low. ADP-dependent phosphorylation of RB60, that might be triggered by the increased pool of ADP during dark growth, can act to reduce'the RNA binding activity of cPABP by enhancing the oxidative catalysis of cPDI over the reductive catalysis, resulting in decreased translation of the *psb*A mRNA. The data presented here show that a PDI such as RB60 acts as a regulator of RNA binding activity and hence gene expression, and not just as a catalyst for protein folding.

The present invention therefore describes the use of a protein disulfide isomerase, such as RB60, to function as a regulator of the binding of a RB47 binding polypeptide to the RB47 binding site nucleotide sequence for the activation of translation. Thus, in view of the foregoing disclosure, the use of a protein disulfide isomerase such as RB60 has many applicabilities in the context of the present invention, particularly ensuring translational control mechanisms for expression of a desired coding sequence and production of the encoded molecule in both oxidizing and reducing environments.

Based on the translational activation mediated through the binding of an RB47 binding site sequence, typically a mRNA sequence and the regulation by the additional element of translational regulator, the present elements of RB47 binding site, an RB47 binding site polypeptide and a RB60 or like molecule are therefore referred to as a regulated translational activation system.

While the invention contemplates the use of any molecule that binds to RB47 binding site and any molecule that functions in accordance to the biological role of RB60 as described herein, in a preferred embodiments, the translational activator protein of RB47 binding site is RB47 and the translation regulatory molecule that regulates the binding of an activator to an RB47 binding site is RB60.

Preferably, a polypeptide that the regulates the binding of a separate polypeptide that binds to a RB47 binding site is present endogenously, i.e., naturally occurring, in a cell such that activation and regulation of translation mediated through a RB47 binding site occurs through an interaction of an endogenous protein with an exogenously provided RB47 binding site sequence as further described in Section C below and in the Examples. Functional RB60 protein is found endogenously found in plant chloroplasts as reviewed above.

In other embodiments, RB60 is a recombinant protein produced through the expression of the coding sequence in a recipient cell as discussed in Section C below. Expression of a recombinant RB60 is now possible in view of the cloning of the cDNA encoding of RB60 as described in the present invention and in Examples 2 and 3. An exemplary recombinant RB60 protein produced by the methods of this invention is more completely described in the Examples.

Thus, although the preferred RB60 protein and nucleic acid compositions are derived from *Chlamydomonas reinhardtii* as discussed in the present invention, variations at both the amino acid and nucleotide sequence level may exist in similar functioning molecules isolated from different algae species as well as within differing plant geni. Such variations are not to be construed as limiting as previously discussed for RB47 compositions.

### C. Recombinant DNA Molecules and Expression Systems that Utilize the RB47 Binding Site

The invention describes several nucleotide sequences of particular use in the methods of controlling gene expression using the RB47 binding site. These sequences include the actual RB47 binding site, the sequences which encode the RB47 protein that binds to the RB47 binding site, the RB60 protein which regulates the-activity of RB47 protein, and various DNA segments, recombinant DNA (rDNA) molecules and vectors constructed for expression of these protein or for using these proteins to control expression of preselected structural genes.

DNA segments of this invention therefore can comprise sequences which encode whole structural genes, fragments of structural genes, and transcription units as described further herein.

A preferred DNA segment is a nucleotide sequence which defines an RB47 binding site as defined herein, which defines an RB47 protein, RB47 polypeptide or biologically active fragment thereof, or which defines an RB60 protein, RB60 polypeptide or biologically active fragment thereof.

The amino acid residue sequence of RB47 and of RB60 are described herein and in the Examples.

A preferred DNA segment codes for an amino acid residue sequence substantially the same as, and preferably consisting essentially of, an amino acid residue sequence or portions thereof corresponding to the RB47 or RB60 protein described herein. Representative and preferred DNA segments are further described in the Examples.

The amino acid residue sequence of a protein or polypeptide is directly related via the genetic code to the deoxyribonucleic acid (DNA) sequence of the structural gene that codes for the protein. Thus, a structural gene or DNA segment can be defined in terms of the amino acid residue sequence, i.e., protein or polypeptide, for which it codes.

An important and well known feature of the genetic code is its redundancy. That is, for most of the amino acids used to make proteins, more than one coding nucleotide triplet (codon) can code for or designate a particular amino acid residue. Therefore, a number of different nucleotide sequences may code for a particular amino acid residue sequence. Such nucleotide sequences are considered functionally equivalent since they can result in the production of the same amino acid residue sequence in all organisms. Occasionally, a methylated variant of a purine or pyrimidine may be incorporated into a given nucleotide sequence. However, such methylations do not affect the coding relationship in any way.

A nucleic acid is any polynucleotide or nucleic acid fragment, whether it be a polyribonucleotide of polydeoxyribonucleotide, i.e., RNA or DNA, or analogs thereof. In preferred embodiments, a nucleic acid molecule is in the form of a segment of duplex DNA, i.e, a DNA segment, although for certain molecular biological methodologies, single-stranded DNA or RNA is preferred.

DNA segments are produced by a number of means including chemical synthesis methods and recombinant approaches, preferably by cloning or by polymerase chain reaction (PCR). DNA segments that encode portions of an RB47 or RB60 protein can easily be synthesized by chemical techniques, for example, the phosphotriester method of Matteucci et al, J. Am. Chem. Soc., 103:3185-3191, 1981, or using automated synthesis methods. In addition, larger DNA segments can readily be prepared by well known methods, such as synthesis of a group of oligonucleotides that define the DNA segment, followed by hybridization and ligation of oligonucleotides to build the complete segment. Alternative methods include isolation of a preferred DNA segment by PCR with a pair of oligonucleotide primers.

Of course, through chemical synthesis, any desired modifications can be made simply by substituting the appropriate bases for those encoding the native amino acid residue sequence.

Furthermore, DNA segments consisting essentially of structural genes encoding an RB47 or RB60 protein can be subsequently modified, as by site-directed or random mutagenesis, to introduce any desired substitutions.

### 1. Cloning RB47 and RB60 Genes

An RB47 or RB60 gene of this invention can be cloned by a variety of cloning methods using *Chlamydomonas reinhardtii (C. reinhardtii*) as a source of the genomic DNA or messenger RNA (mRNA) for cloning purposes. Cloning these genes can be conducted according to the general methods described in the Examples.

Preferred cloning strategies for isolating a nucleic acid molecule that encodes an RB47 or RB60 protein of this invention are described in the Examples.

Sources of libraries for cloning an RB47 or RB60 gene of this invention can include genomic DNA or messenger RNA (mRNA) in the form of a cDNA library from a tissue believed to express these proteins. The preferred tissue is plant chloroplast from *C. reinhardtii.*

A preferred cloning method involves the preparation a *C. reinhardtii* chloroplast cDNA library using standard methods, and preparing the RB47 or RB60-encoding nucleotide sequence using PCR with oligonucleotide primers based on the nucleotide sequences described herein for the RB47 or RB60 genes, respectively. Alternatively, the desired cDNA clones can be identified and isolated from a cDNA or genomic library by conventional hybridization methods using a hybridization probe based on the sequences described herein. Other methods are readily apparent to one skilled in the art.

### 2. Expression Vectors

In addition, the invention contemplates a recombinant DNA molecule (rDNA) containing a DNA segment of this invention encoding an RB47 or RB60 protein as described herein. A rDNA can be produced by operatively (operably) linking a vector to a DNA segment of the present invention.

As used herein, the term "vector" refers to a DNA molecule capable of autonomous replication in a cell and to which another DNA segment can be operatively linked so as to bring about replication of the attached segment. A vector adapted for expression of a gene product and capable of directing the expression of a structural gene is referred to herein as an "expression vector". Thus, a recombinant DNA molecule is a hybrid DNA molecule comprising at least two nucleotide sequences not normally found together in nature.

The choice of vector to which a DNA segment of the present invention is operatively linked depends directly, as is well known in the art, on the functional properties desired, e.g., protein expression, and the host cell to be transformed, these being limitations inherent in the art of constructing recombinant DNA molecules. However, a vector contemplated by the present invention is at least capable of directing the replication, and preferably also expression, of a structural gene included in DNA segments to which it is operatively linked.

Both prokaryotic and eukaryotic expression vectors are familiar to one of ordinary skill in the art of vector construction and are described by Ausebel, et al., In Current Protocols in Molecular Biology, Wiley and Sons, New York (1993) and by Sambrook et al., Molecular Cloning: A Laboratory Manual, Cold Spring Harbor Laboratory, (1989), which reference also describes all the general recombinant DNA methods referred to herein.

In one embodiment, a vector contemplated by the present invention includes a procaryotic replicon, i.e., a DNA sequence having the ability to direct autonomous replication and maintenance of the recombinant DNA molecule extrachromosomally in a procaryotic host cell, such as a bacterial host cell, transformed therewith. Such replicons are well known in the art. In addition, those embodiments that include a procaryotic replicon also include a gene whose expression confers drug resistance to a bacterial host transformed therewith. Typical bacterial drug resistance genes are those that confer resistance to ampicillin or tetracycline.

Those vectors that include a procaryotic replicon can also include a procaryotic promoter capable of directing the expression (transcription and translation) of a structural gene in a bacterial host cell, such as E. coli, transformed therewith. A promoter is an expression control element formed by a DNA sequence that permits binding of RNA polymerase and transcription to occur. Promoter sequences compatible with bacterial hosts are typically provided in plasmid vectors containing convenient restriction sites for insertion of a DNA segment of the present invention. Typical of such vector plasmids are pUC8, pUC9, pBR322 and pBR329 available from Biorad Laboratories, (Richmond, CA), pRSET available from Invitrogen (San Diego, CA) and pPL and pKK223 available from Pharmacia, Piscataway, N.J.

Expression vectors compatible with eucaryotic cells, preferably those compatible with vertebrate cells, can also be used to form the recombinant DNA molecules of the present invention. Eucaryotic cell expression vectors are well known in the art and are available from several commercial sources. Typically, such vectors are provided containing convenient restriction sites for insertion of the desired DNA segment. Typical of such vectors are pSVL and pKSV-10 (Pharmacia), pBPV-1/pML2d (International Biotechnologies, Inc.), pTDT1 (ATCC, #31255), pRc/CMV (Invitrogen, Inc.), the preferred vector pcDNA3 (Invitrogen) described in the Examples, and the like eucaryotic expression vectors.

An alternative expression system that can be used to express a protein of the invention is an insect system. In one such system, Autographa californica nuclear polyhidrosis virus (AcNPV) is used as a vector to express foreign genes. The virus grows in Spodoptera frugiperda cells. The polypeptide-encoding nucleotide sequence may be cloned into non-essential regions (in Spodoptera frugiperda for example the polyhedron gene) of the virus and placed under control of an AcNPV promoter (for example the polyhedron promoter). Successful insertion of the polypeptide-encoding nucleotide sequence inactivates the polyhedron gene and production of non-occluded recombinant virus (i.e., virus lacking the proteinaceous coat coded for by the polyhedron gene). These recombinant viruses are then used to infect cells in which the inserted gene is expressed. See Smith et al., J. Biol. Chem., 46:584 (1983); and Smith, U.S. Patent No. 4,215,051.

Mammalian cell systems that utilize recombinant viruses or viral elements to direct expression may be engineered. For example, when using adenovirus expression vectors, the coding sequence of a polypeptide may be ligated to an adenovirus transcription/translation control complex, e.g., the late promoter and tripartite leader sequence. This chimeric gene may then be inserted into the adenovirus genome by in vitro or in vivo recombination. Insertion in a non-essential region of the viral genome (e.g., region E1 or E3) will result in a recombinant virus that is viable and capable of expressing the polypeptide in infected hosts (e.g., see Logan et al., Proc. Natl. Acad. Sci., USA, 81:3655-3659 (1984)). Alternatively, the vaccinia virus 7.5K promoter may be used. (e.g., see, Mackett et al., Proc. Natl. Acad. Sci., USA, 79:7415-7419 (1982); Mackett et al., J. Virol., 49:857-864 (1984); Panicali et al., Proc. Natl. Acad. Sci.. USA, 79:4927-4931 (1982)). Of particular interest are vectors based on bovine papilloma virus which have the ability to replicate as extrachromosomal elements (Sarver et al., Mol. Cell. Biol., 1:486 (1981)). Shortly after entry of this DNA into mouse cells, the plasmid replicates to about 100 to 200 copies per cell. Transcription of the inserted cDNA does not require integration of the plasmid into the host's chromosome, thereby yielding a high level of expression. These vectors can be used for stable expression by including a selectable marker in the plasmid, such as the neo gene. Alternatively, the retroviral genome can be modified for use as a vector capable of introducing and directing the expression of the polypeptide-encoding nucleotide sequence in host cells ( Cone et al., Proc Natl Acad Sci., USA, 81:6349-6353 (1984)). High level expression may also be achieved using inducible promoters, including, but not limited to, the metallothionine IIA promoter and heat shock promoters.

For long-term, high-yield production of recombinant proteins, stable expression is preferred. Rather than using expression vectors which contain viral origins of replication, host cells can be transformed with a cDNA controlled by appropriate expression control elements (e.g., promoter and enhancer sequences, transcription terminators, polyadenylation sites, etc.), and a selectable marker. As mentioned above, the selectable marker in the recombinant plasmid confers resistance to the selection and allows cells to stably integrate the plasmid into their chromosomes and grow to form foci which in turn can be cloned and expanded into cell lines.

For example, following the introduction of foreign DNA, engineered cells may be allowed to grow for 1-2 days in an enriched media, and then are switched to a selective media. A number of selection systems may be used, including but not limited to the herpes simplex virus thymidine kinase (Wigler et al., Cell, 11:223 (1977)), hypoxanthine-guanine phosphoribosyltransferase (Szybalska et al, Proc. Natl. Acad. Sci., USA, 48:2026 (1962)), and adenine phosphoribosyltransferase (Lowy et al., Cell, 22:817 (1980)) genes, which can be employed in tk⁻, hgprt⁻ or aprt⁻ cells respectively. Also, antimetabolite resistance-conferring genes can be used as the basis of selection; for example, the genes for dhfr, which confers resistance to methotrexate ( Wigler et al., Proc. Natl. Acad. Sci.. USA, 77:3567 (1980); O'Hare et al., Proc. Natl. Acad. Sci., USA, 78:1527 (1981); gpt, which confers resistance to mycophenolic acid (Mulligan et al, Proc. Natl. Acad. Sci., USA, 78:2072, (1981)); neo, which confers resistance to the aminoglycoside G-418 (Colberre-Garapin et al, J. Mol. Biol., 150:1 (1981)); and hygro, which confers resistance to hygromycin (Santerre et al, Gene, 30:147 (1984)). Recently, additional selectable genes have been described, namely trpB, which allows cells to utilize indole in place of tryptophan; hisD, which allows cells to utilize histinol in place of histidine (Hartman et al, Proc. Natl. Acad. Sci.. USA, 85:804 (1988)); and ODC (ornithine decarboxylase) which confers resistance to the ornithine decarboxylase inhibitor, 2-(difluoromethyl)-DL-ornithine, DFMO (McConlogue L., In: Current Communications in Molecular Biology, Cold Spring Harbor Laboratory ed., (1987)).

In another preferred embodiment, expression vectors compatible for use with plant cells are used to express structural genes in plants. Plants provide advantageous expression and delivery aspects in that a large supply of bulk protein with universal access is readily made from which the protein is either isolatable therefrom. Thus, transgenic plants containing expression vectors for encoding a recombinant protein of this invention is useful for preparing polypeptides of this invention.

Typical expression vectors useful for expression of genes in plants are well known in the art. Typical methods for introducing genes via expression vectors into plants include Agrobacterium tumefaciens-mediated transformation, plant virus transfection, protoplast transformation, gene transfer into pollen, injection into reproductive organs, injection into immature embryos, and direct insertion, a process referred to as "biolystics". In the case of infection by plant viruses, a recombinant protein can be produced at high concentrations and isolated at low cost, with the genetic stocks being easily maintained for long periods of time without passaging through plants.

Preferred plants for such expression include any plant for which a compatible expression vector system exists, including dicots and monocots. Particularly preferred plants include alfalfa, tomato, petunia, soy bean, tobacco, corn, wheat, rice, spinach, asparagus, and the like. Exemplary plant expression vector systems for expression of a recombinant protein of this invention include those, such as binary vector system utilizing Agrobacterium tumefaciens, described in US Patent Number 5,202,422 and An et al., Plant Molecular Biology Manual, A3:1-19 (1988). Additional plant expression systems are described in US Patent Numbers 5,234,834, the disclosures of which several teachings are hereby incorporated by reference.

### D. Methods

The present invention provides for a variety of methods using the disclosed recombinant proteins, nucleotide sequences, expression cassettes and expression systems. In particular, the invention provides methods for preparing (expressing) an RB47 or RB60 protein using the expression systems, methods for controlling (regulating) the expression of a preselected coding sequence using the translation regulation properties of the RB47 system, methods for screening for useful agents which affect the ability of RB47 and/or RB60 to regulate translation in these systems, and the like methods.

### 1. Methods for Preparing a Recombinant Protein

An RB47 OR RB60 protein of this invention can be prepared by a variety of means, although expression using a rDNA expression vector is preferred. Exemplary production methods for a recombinant protein are described in the Examples.

In one embodiment, the invention contemplates methods for the preparation of a recombinant RB47 or RB60 proteins in their various forms using a nucleotide sequence-based expression system. The produced proteins are useful in the various embodiments described herein.

Although the description of expression is limited to specific examples, it is to be understood that the expression of proteins is generally characterized, and the expression of, for example, full length RB47 protein serves as an example of expression of any of a variety of forms of RB47 protein, including mature RB47, processed forms of RB47, biologically active fragments of RB47, fusion proteins containing RB47 domains, and the like. The descriptions herein apply to various forms of RB60 as well.

The method of preparing a recombinant RB47 or RB60 protein comprises providing an expression cassette as described herein that contains nucleotide sequences that encode an RB47 or RB60 protein, or fragment thereof, together with nucleotide sequences that provide the requisite information for controlling gene expression and translation. The provided cassette is introduced into a suitable expression medium and maintained under conditions and for a time period sufficient for expression and translation of the protein product to occur. The times and conditions can vary, as is well known, depending upon the expression/translation medium (e.g., intracellular medium, in vitro expression medium, etc.). Nucleotide sequence information required for expression and translation are also well known in the art and need not be described in detail herein.

A typical expression system is described herein, in which the expression cassette is present on a recombinant plasmid that has been introduced into a microbial host. For example, the expression cassette is present in a PET expression plasmid introduced into *E. coli*, and the transformed bacterial is cultivated under growth conditions suitable for growth and expression of the expression cassette. Additional expression systems include other species of bacterial cells, yeast and eucaryotic cells, including mammalian cell expression systems, and in vitro expression systems, as are well known.

After expression, the expressed RB60 or RB47 protein is readily isolated from the expression medium (i.e., the host cell and cell contents) using standard biochemical separation methods to produce an isolated recombinant protein. Typical isolation methods can include disruption of the cell followed by protein fractionations using mechanical, chemical, biological or immunological properties of the RB47 or RB60 protein. Preferred separation/isolation methods are described in the Examples.

Thus, the invention also provides a method for the production of recombinant proteins, either as intact RB47 or RB60 protein, as fusion proteins or as smaller polypeptide fragments of RB47 or RB60. The production method generally involves inducing cells to express a recombinant protein of this invention, recovering the expressed protein from the resulting cells, and purifying the expressed protein so recovered by biochemical fractionation methods, using a specific antibody of this invention, or other chemical procedures. Inducing expression of a recombinant protein can comprise inserting a rDNA vector encoding an RB47 or RB60-protein, or fragment thereof, of this invention, which rDNA is capable of expressing the structural gene encoding the RB47 or RB60 protein, into a suitable host cell, and expressing the vector's structural gene.

Thus, to facilitate expression of a recombinant protein or fusion protein of the present invention, DNA segments encoding either RB47 or RB60 as described herein, or portions thereof, are inserted into an expression vector. DNA segments are characterized as including a DNA sequence that encodes a recombinant protein of this invention, i.e., RB47 or RB60. That is, the DNA segments of the present invention are characterized by the presence of some or all of an RB47 or RB60 structural gene as described herein. Preferably the gene is present as an uninterrupted linear series of codons where each codon codes for an amino acid residue found in the native protein, i.e., a gene free of introns.

### 2. Translational Regulation of Expression of a Coding Sequence

In a related embodiment, the invention contemplates methods for the controlled expression of a preselected coding sequence under the regulation of RB47 or a combination of RB47 and RB60 using the nucleotide sequences described herein that define an RB47 binding site and the recombinant proteins which bind this binding site and regulate translation of adjacent nucleotide sequences.

Thus, for example, the RB47 binding site can be engineered into an expression cassette as described herein to control the expression of a structural gene nucleotide sequence which encodes a preselected gene. The expression cassette contains the following genetic elements: (1) a promoter sequence that initiates transcription of a gene, (2) an RB47 binding site sequence adjacent to and located 3' relative to the promoter sequence, (3) a structural protein coding sequence under the expression control of the promoter, and a source of RB47 protein to regulate the expression of the cassette.

The promoter can be any of a variety of genetic elements as are well known for promotion of gene transcription. The promoter can be constitutive, inducible or repressible, thereby providing further regulation. A preferred promoter is the lac z promoter inducible by IPTG, as is well known. Additional promoters include the T3 or T7 promoter.

The RB47 protein can be provided exogenously, as by addition of isolated protein to an expression medium containing the cassette, provided endogenously, as by introducing the cassette into a host cell which contains endogenous RB47 (e.g., a chloroplast cell that expresses RB47) or provided by introducing a gene which expresses the RB47 protein into the expression medium, either in combination with the cassette, or substantially contemporaneously with the cassette. The RB47 encoding gene can be added as a separate plasmid, or can be present as a second translation unit on the cassette which expresses the preselected gene.

Thus, in one embodiment, the invention comprises first forming an expression cassette by operably linking the above-identified components, and then introducing the expression cassette into a cell or other suitable expression medium.

Where the expression system can be further regulated by RB60, the RB60 protein can be added to the expression system exogenously from purified recombinant protein, provided as an endogenous protein when expression is carried out in a plant cell, or can be provided by expression from a second translation unit. The second transcription unit can be present on a separate nucleotide sequence, such as a separate plasmid capable of expressing RB60 that contains the RB60 nucleotide sequence, or present on the same expression cassette as a separate translation unit for RB60.

Expression cassettes can be introduced into an expression medium by any of a variety of means, and therefore the invention need not be so limited. For example, a variety of cell types can be used including bacterial, plant, yeast and higher eukaryote, all of which have different methods for transformation, including transduction, transfection, electroporation, transformation, biolistic bombardment, infection, and the like.

These systems provide particular advantages in the expression of preselected genes, including structural genes, insofar as these systems provide the ability to control timing and amounts of expression by specific and strong regulators of translation. The advantages will be apparent to one skilled in the art, but include synchronized expression in cell populations, combining expression with nutrient supplementation, regulated expression in therapeutic, manufacturing and diagnostic expression applications, and the like systems.

In one embodiment, the method for expression of a desired (preselected) coding sequence comprises first the method of preparing an expression cassette having the various components described herein, followed by introducing the cassette into an expression medium and maintaining the cassette under condition suitable for expression. To that end, the cassette can be prepared by any recombinant DNA method, which methods are well established in the art, including use of restriction enzymes to ligate nucleotide fragments, polymerase chain reactions (PCR) to isolate, mutate, modify and manipulate nucleotide fragments, and cloning sites for insertion of preselected genes. An exemplary method involves operably linking the RB47 binding site sequence to a cloning site for insertion of a desired coding sequence, such that the cloning site is downstream of the binding site, and linking a second nucleotide sequence which encodes an RB47 polypeptide. The method can further involve linking a promoter 5' upstream to the RB47 binding site to form a transcription unit containing from 5' to 3' a promoter, a binding site and a cloning site for inserting the desired coding sequence. In a subsequent step for forming an expression cassette, the desired coding sequence is inserted into the cloning site.

Other permutations will be apparent to one skilled in the art.

### 3 Screening for Agonists and Antagonists of RB47-Mediated Translation

In another embodiment, the invention contemplates using an expression cassette containing an RB47 binding site to screen for agonists and antagonists which affect RB47 binding to the RB47 binding site, thereby identifying useful reagents for further control of an RB47-regulated (mediated) translation unit.

The method comprises providing an expression cassette according to the invention and having a indicator polypeptide as the desired structural gene into an expression system (i.e., medium), introducing RB47 and the candidate agent, and detecting the amount of indicator polypeptide expressed, and thereby the amount of effect the agent has on the expression system. Controls are typically run in the presence and absence of the RB47 protein to demonstrate selectivity of the agent, which could be either an agonist or antagonist of RB47 activation of translation upon binding to the RB47 binding site.

Typical indicator polypeptides include enzymes which produce detectable substrates, light producing enzymes, such as luciferase, and the like. The RB47 can be added in the form of exogenous protein or by expression off of a nucleotide sequence, as described earlier.

In one embodiment, the expression system is a cell capable of supporting expression (transcription and translation) and the RB47 is provided in the cell either by adding protein to the cell or by providing a RB47-encoding nucleotide sequence to the cell.

In a further embodiment, the screening method is useful to identify agonists or antagonists of RB60 or RB60-mediated regulation of RB47-mediated translation, ie., reagents which effect RB60 rather than RB47 directly. This embodiment requires that the additional component RB60 be included in the screening method as described herein for expression using RB60.

Additional permutations are readily apparent to one skilled in the art.

### E. Articles of Manufacture

The present invention also contemplates an article of manufacture comprising one or more of the components of the present invention. Typically, the article is present in the form of a package containing the component or in combination with packaging material. The packaging material includes a label or instructions for use of the components of the package. The instructions indicate the contemplated use of the component as described herein for the methods or compositions of the invention.

For example, an article of manufacture can comprise one or both of the recombinant proteins of the invention, RB47 and RB60, in amounts useful in a method according to the invention. Alternatively, an article of manufacture can contain an expression cassette for expressing a desired coding sequence, which cassette comprises a construction as described herein that includes an RB47 binding site, and can optionally include a cloning site for insertion of a desired coding sequence, a promoter for controlling transcription of the expression cassette and inserted coding sequence, a coding sequence for the RB47 protein and/or the RB60 protein, and a preselected coding sequence. Alternatively, the article of manufacture may contain multiple nucleotide sequences, such as separate plasmid each encoding a different transcription, comprising one or more of the desired coding sequence under control of the RB47 binding site, the RB47 coding sequence and the RB60 coding sequence.

The article of manufacture may optionally include both an expression cassette and one or both of the recombinant proteins RB47 and RB60, or may contain a cell transformed by one or more of the expression cassettes of the present invention.

In a related embodiment, an expression cassette may be used for expressing an RNA transcript containing an RB47 binding site, useful for subsequent regulation of translation of the transcript by RB47 protein. Such a construct can be used in the RNA expression field. Therefore, the invention contemplates an article of manufacture comprising packaging material, and in a separate container an expression cassette for expressing RNA that includes the RB47 binding site, wherein the packaging material includes a label that indicates the uses of the cassette in producing in vitro RNA transcripts. The production of RNA transcripts is well known. The article can further contain in separate containers components useful in combination with the cassette, including polymerases buffers, ribonucleotides and other reagents for *in vitro* transcription.

In these permutations, the components may optionally be present in the article of manufacture in separate containers.

### Examples

The following examples relating to this invention are illustrative and should not, of course, be construed as specifically limiting the invention. Moreover, such variations of the invention, now known or later developed, which would be within the purview of one skilled in the art are to be considered to fall within the scope of the present invention hereinafter claimed.

### 1. Cloning of RB47

RB47 protein, 47 kilodalton (kDa) was purified by published procedures ( Danon and Mayfield, Embo J., 10:3993-4001 (1991)). The protein was then digested with proteinase Lys-C or trypsin, and the peptides separated HPLC and microsequenced (John Lesyk, Worchester Foundation for Experimental Biology, Worcester, MA and Arie Admon, The Protein Center, Department of Biology, Technion, Haifa, Israel). Two peptide sequences were obtained (QYGFVHFEDQAAADR (SEQ ID NO 1) and GFGFINFKDAESAA (SEQ ID NO 2)). Degenerate oligonucleotides were designed based on the reverse translation of these peptides. For the QYG... and GFG... peptides, the respective oligonucleotide sequences were 5'CAGTACGGYTTCGTBCAYTTCGAGGAYCAGGC3' (SEQ ID NO 3) and 5'GGAATTCGGYTTCGGYTTCATYAACTTCAAGGAYGCBGAG3' (SEQ ID NO 4), where the underline indicates an Eco RI restriction site and where Y=C or T; and B=G or T or C. A *C. reinhardtii* cDNA λ-gt10 phage library obtained from EMBL Laboratories, Heidelberg, Germany, was screened with these oligonucleotides using standard methods as described by Sambrook et al., Molecular Cloning: A Laboratory Manual, Cold Spring Harbor Laboratory Press, Chapter 8, 1989. One set of duplicate filter lifts was probed with each oligonucleotide, and plaques that hybridized to both were isolated. Several cDNA clones that hybridized to oligonucleotides from both peptides were identified. Four of these clones were 2.6 kilobases (kb) in length, the predicted full length of the RB47 mRNA. One of these cDNAs was subcloned into an *E. coli* plasmid for sequence determination using an automated sequencer.

The nucleotide and encoded amino acid sequence of RB47 is also shown in Figures 1A-1D (SEQ ID NO 5). As described in Section 2 above, the predicted protein sequence from the cloned cDNA contained both the derived peptide sequences of RB47 and is highly homologous to poly(A) binding proteins (PABP) from a variety of eukaryotic organisms.

### 2. Cloning of RB60

To clone the cDNA encoding the 60 kDa *psb*A mRNA binding protein (RB60), the *psb*A-specific RNA binding proteins were purified from light-grown *C. reinhardtii* cells using heparin-agarose chromatography followed by *psbA* RNA affinity chromatography (RAC). RAC-purified proteins were separated by two-dimensional polyacrylamide gel electrophoresis. The region corresponding to RB60 was isolated from the PVDF membrane. RB60 protein was then digested with trypsin. Unambiguous amino acid sequences were obtained from two peptide tryptic fragments (WFVDGELASDYNGPR (SEQ ID NO 6) and (QLILWTTADDLKADAEIMTVFR (SEQ ID NO 7)) as described above for RB47. The calculated molecular weights of the two tryptic peptides used for further analysis precisely matched with the molecular weights determine by mass spectrometry. The DNA sequence corresponding to one peptide of 22 amino acid residues was amplified by PCR using degenerate oligonucleotides, the forward primer 5'CGCGGATCCGAYGCBGAGATYATGAC3' (SEQ ID NO 8) and the reverse primer 5'CGCGAATTCGTCATRATCTCVGCRTC3' (SEQ ID NO 9), where R can be A or G (the other IUPAC nucleotides have been previously defined above). The amplified sequence was then used to screen a λ-gt10 cDNA library from *C. reinhardtii*. Three clones were identified with the largest being 2.2 kb. Selection and sequencing was performed as described for RB47 cDNA.

The resulting RB60 cDNA sequence is available via GenBank (Accession Number AF027727). The nucleotide and encoded amino acid sequence of RB60 is also shown in Figures 2A-2B (SEQ ID NO 10). The protein coding sequence of 488 amino acid residues corresponds to nucleotide positions 16-1614 of the 2413 base pair sequence. The predicted amino acid sequence of the cloned cDNA contained the complete amino acid sequences of the two tryptic peptides. The amino acid sequence of the encoded protein revealed that it has high sequence homology to both plant and mammalian protein disulfide isomerase (PDI), and contains the highly conserved thioredoxin-like domains with -CysGlyHisCys- (-CGHC-) (SEQ ID NO 11) catalytic sites in both the N-terminal and C-terminal regions and the -LysAspGluLeu- (-KDEL-) (SEQ ID NO 12) endoplasmic reticulum (ER) retention signal at the C-terminus found in all PDIs. PDI is a mutifunctional protein possessing enzymatic activities for the formation, reduction, and isomerization of disulfide bonds during protein folding, and is typically found in the ER. The first 30 amino acid residues of RB60 were found to lack sequence homology with the N-terminal signal sequence of PDI from plants or mammalian cells. However, this region has characteristics of chloroplast transit peptides of *C. reinhardtii*, which have similarities with both mitochondrial and higher plant chloroplast presequences. A transit peptide sequence should override the function of the -KDEL- ER retention signal and target the protein to the chloroplast since the -KDEL- signal acts only to retain the transported protein in the ER.

### 3. Preparation of psbA Promoter Sequence and RB47 Binding Site Nucleotide Sequence

The chloroplast *psb*A gene from the green unicellular alga *C. reinhardii* was cloned and sequenced as described by Erickson et al., Embo J., 3:2753-2762 (1984), the disclosure of which is hereby incorporated by reference. The DNA sequence of the coding regions and the 5' and 3' untranslated (UTR) flanking sequences of the *C. reinhardii psb*A gene is shown in Figures 3A-3C. The *psb*A gene sequence is also available through GenBank as further discussed in Example 4. The nucleotide sequence is also listed as SEQ ID NO 13. The deduced amino acid sequence (also listed in SEQ ID NO 13) of the coding region is shown below each codon beginning with the first methionine in the open reading frame. Indicated in the 5' non-coding sequence are a putative Shine-Dalgarno sequence in the dotted box, two putative transcription initiation sites determined by S1 mapping (S1) and the Pribnow-10 sequence in the closed box. Inverted repeats of eight or more base pairs are marked with arrows and labeled A-D. A direct repeat of 31 base pairs with only two mismatches is marked with arrows labeled 31. Indicated in the 3' non-coding sequence is a large inverted repeat marked by a forward arrow and the SI cleavage site marking the 3' end of the mRNA. Both the 5' and 3' untranslated regions are used in preparing one of the expression cassettes of this invention as further described below.

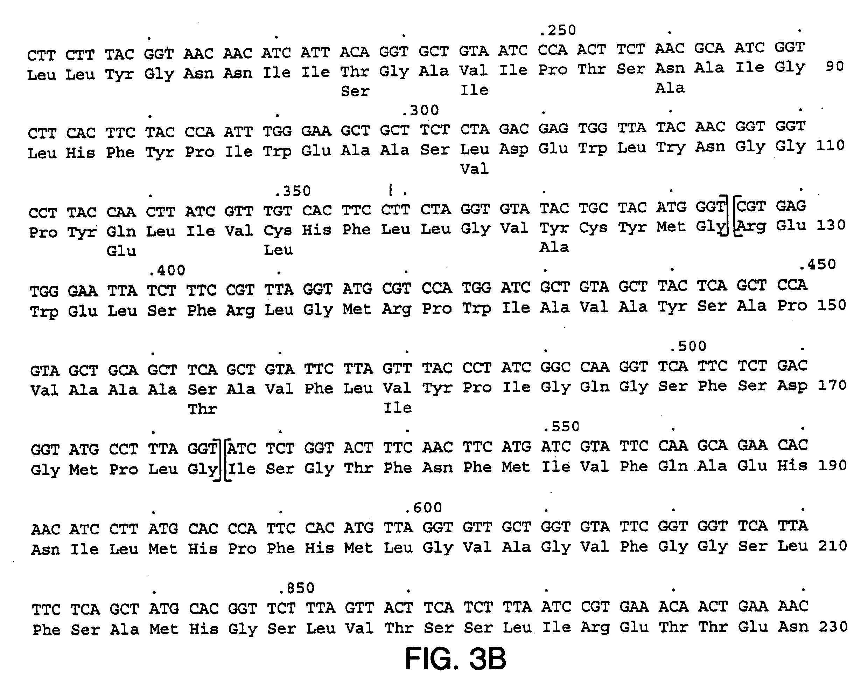

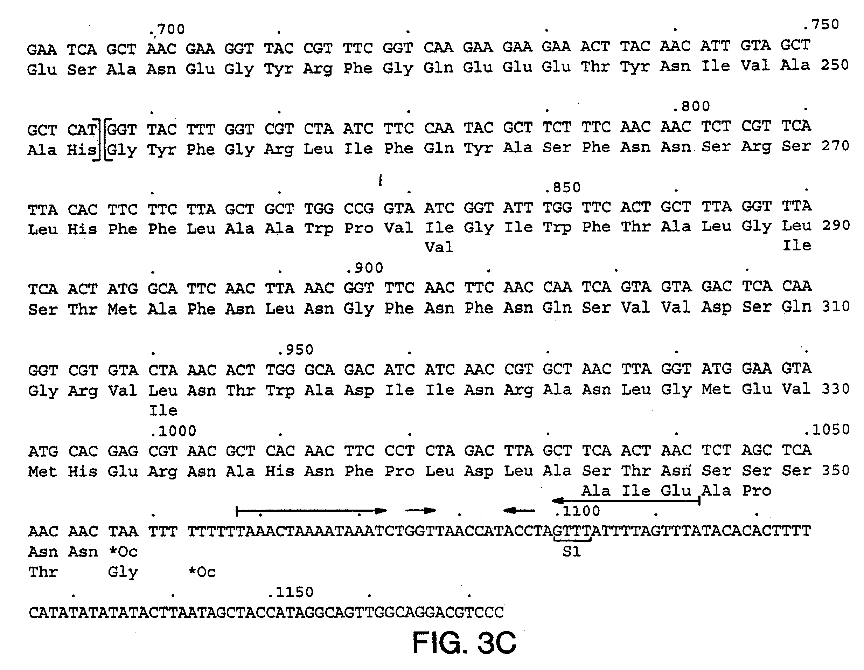

The 5' UTR as previously discussed contains both the *psb*A promoter and the RB47 binding site. The nucleotide sequence defining the *psb*A promoter contains the region of the *psb*A DNA involved in binding of RNA polymerase to initiate transcription. The -10 sequence component of the *psb*A promoter is indicated by the boxed nucleotide sequence upstream of the first S1 while the -35 sequence is located approximately 35 bases before the putative initiation site. As shown in Figure 3, the -10 sequence is boxed, above which is the nucleotide position (-100) from the first translated codon. The -35 sequence is determined accordingly. A *psb*A promoter for use in an expression cassette of this invention ends at the first indicated S1 site (nucleotide position -92 as counting from the first ATG) in Figure 3 and extends to the 5' end (nucleotide position -251 as shown in Figure 3). Thus, the promoter region is 160 bases in length. A more preferred promoter region extends at least 100 nucleotides to the 5' end from the S1 site. A most preferred region contains nucleotide sequence ending at the s1 site and extending 5' to include the -35 sequence, i.e., from -92 to -130 as counted from the first encoded amino acid residue (39 bases).

The *psb*A RB47 binding site region begins at the first S1 site as shown in Figure 3 and extends to the first adenine base of the first encoded methionine residue. Thus, a *psb*A RB47 binding site in the *psb*A gene corresponds to the nucleotide positions from -91 to -1 as shown in Figure 3.

The above-identified regions are used to prepare expression constructs as described below. The promoter and RB47 binding site regions can be used separately; for example, the RB47 binding site sequence can be isolated and used in a eukaryotic or prokaryotic plasmid with a non-*psb*A promoter. Alternatively, the entire *psb*A 5' UTR having 251 nucleotides as shown in Figure 3 is used for the regulatory region in an expression cassette containing both the *psb*A promoter and RB47 binding site sequence as described below.

### 4. Preparation of Expression Vectors and Expression of coding Sequences

### A. Constructs Containing an psbA Promoter, an RB47 Binding Site Nucleotide Sequence, a Desired Heterologous Coding Sequence, an RB47-Encoding Sequence and an RB60-Encoding Sequence

Plasmid expression vector constructs, alternatively called plasmids, vectors, constructs and the like, are constructed containing various combinations of elements of the present invention as described in the following examples. Variations of the positioning and operably linking of the genetic elements described in the present invention and in the examples below are contemplated for use in practicing the methods of this invention. Methods for manipulating DNA elements into operable expression cassettes are well known in the art of molecular biology. Accordingly, variations of control elements, such as constitutive or inducible promoters, with respect to prokaryotic or eukaryotic expression systems as described in Section C. are contemplated herein although not enumerated. Moreover, the expression the various elements is not limited to one transcript producing one mRNA; the invention contemplates protein expression from more than one transcript if desired.

As such, while the examples below recite one or two types of expression cassettes, the genetic elements of RB47 binding site, any desired coding sequence, in combination with RB47 and RB60 coding sequences along with a promoter are readily combined in a number of operably linked permeations depending on the requirements of the cell system selected for the expression. For example, for expression in a chloroplast, endogenous RB47 protein is present therefore an expression cassette having an RB47 binding site and a desired coding sequence is minimally required along with a operative promoter sequence. Overexpression of RB47 may be preferable to enhance the translation of the coding sequence; in that case, the chloroplast is further transformed with an expression cassette containing an RB47-encoding sequence. Although the examples herein and below utilize primarily the sequence encoding the precursor form of RB47, any of the RB47-encoding sequences described in the present invention, i.e., RB47 precursor, mature RB47 and histidine-modified RB47 are contemplated for use in any expression cassette and system as described herein. To regulate the activation of translation, an RB60-encoding element is provided to the expression system to provide the ability to regulate redox potential in the cell as taught in Section B. These examples herein and below represent a few of the possible permutations of genetic elements for expression in the methods of this invention.

In one embodiment, a plasmid is constructed containing an RB47 binding site directly upstream of an inserted coding region for a heterologous protein of interest, and the RB47 and RB60 coding regions. Heterologous refers to the nature of the coding region being dissimilar and not from the same gene as the regulatory molecules in the plasmid, such as RB47 and RB60. Thus, all the genetic elements of the present invention are produced in one transcript from the IPTG-inducible *psb*A promoter. Alternative promoters are similarly acceptable.

The final construct described herein for use in a prokaryotic expression system makes a single mRNA from which all three proteins are translated. The starting plasmid is any *E. coli* based plasmid containing an origin of replication and selectable marker gene. For this example, the Bluescript plasmid, pBS, commercially available through Stratagene, Inc., La Jolla, CA, which contains a polylinker-cloning site and an ampicilin resistant marker is selected for the vector.

The wild-type or native *psb*A gene (Erickson et al., Embo J., 3:2753-2762 (1984), also shown in Figure 3, is cloned into pBS at the EcoRI and BamHI sites of the polylinker. The nucleotide sequence of the *psb*A gene is available on GenBank with the 5' UTR and 3' UTR respectively listed in Accession Numbers X01424 and X02350. The EcoRI site of *psb*A is 1.5 kb upstream of the *psb*A initiation codon and the BamHI site is 2 kb downstream of the stop codon. This plasmid is referred to as pDl.

Using site-directed PCR mutagenesis, well known to one of ordinary skill in the art, an NdeI site is placed at the initiation codon of *psb*A in the pDl plasmid so that the ATG of the NdeI restriction site is the ATG initiation codon. This plasmid is referred to as pDl/Nde. An Nde site is then placed at the initiation codon of the gene encoding the heterologous protein of interest and an Xho I site is placed directly downstream (within 10 nucleotides) of the TAA stop codon of the heterologous protein coding sequence. Again using site-directed mutagenesis, an XhoI site is placed within 10 nucleotides of the initiation codon of RB47, the preparation of which is described in Example 2, and an NotI site is placed directly downstream of the stop codon of RB47. The heterologous coding region and the RB47 gene are then ligated into pDl/Nde so that the heterologous protein gene is directly adjacent to the RB47 binding site and the RB47 coding region is downstream of the heterologous coding region, using the Xho I site at the heterologous stop codon and the Not I site of the pDl polylinker.

These genetic manipulations result in a plasmid containing the 5' end of the *psb*A gene including the promoter region and with the RB47 binding site immediately upstream of a heterologous coding region, and the RB47 coding region immediately downstream of the heterologous coding region. The nucleotides between the stop codon of the heterologous coding region and the initiation codon of the RB47 coding region is preferably less than 20 nucleotides and preferably does not contain any additional stop codons in any reading frame. This plasmid is referred to as pDl/RB47.

Using site-directed mutagenesis, a NotI site is placed immediately (within 10 nucleotides) upstream of the initiation codon of RB60, the preparation of which is described in Example 2, and an Xba I site is placed downstream of the RB60 stop codon. This DNA fragment is then ligated to the 3' end of the *psb*A gene using the Xba I site found in the 3' end of the *psb*A gene so that the *psb*A 3' end is downstream of the RB60 coding region. This fragment is then ligated into the pD1/RB47 plasmid using the NotI and BamHI sites so that the RB60 coding region directly follows the RB47 coding region. The resulting plasmid is designated pD1/RB47/RB60. Preferably there is less then 20 nucleotides between the RB47 and RB60 coding regions and preferably there are no stop codons in any reading frame in that region. The final plasmid thus contains the following genetic elements operably linked in the 5' to 3' direction: the 5' end of the *psbA* gene with a promoter capable of directing transcription in chloroplasts, an RB47 binding site, a desired heterologous coding region, the RB47 coding region, the RB60 coding region, and the 3' end of the *psb*A gene which contains a transcription termination and mRNA processing site, and an *E. coli* origin of replication and amplicillin resistance gene. A diagram of this plasmid with the restriction sites is shown in Figure 4.

Expression of pD1/RB47/RB60 in *E. coli* to produce recombinant RB47, RB60 and the recombinant heterologous protein is performed as described in Example 4B. The heterologous protein is then purified as further described.

Expression cassettes in which the sequences encoding RB47 and RB60 are similarly operably linked to a heterologous coding sequence having the *psb*A RB47 binding site as described in Example 3 are prepared with a different promoter for use in eukaryotic, such as mammalian expression systems. In this aspect, the cassette is similarly prepared as described above with the exception that restriction cloning sites are dependent upon the available multiple cloning sites in the recipient vector. Thus, the RB47 binding site prepared in Example 3 is prepared for directed ligation into a selected expression vector downstream of the promoter in that vector. The RB47 and RB60 coding sequences are obtained from the pD1/RB47/RB60 plasmid by digestion with XhoI and XbaI and inserted into a similarly digested vector if the sites are present. Alternatively, site-directed mutagenesis is utilized to create appropriate linkers. A desired heterologous coding sequence is similarly ligated into the vector for expression.

### B. Constructs Containing RB47 Nucleotide Sequence

### 1) Purified Recombinant RB47 Protein

In one approach to obtain purified recombinant RB47 protein, the full length RB47 cDNA prepared above was cloned into the *E. coli* expression vector pET3A (Studier et al., Methods Enzymol., 185:60-89 (1990)), also commercially available by Novagen, Inc., Madison, WI and transformed into BL21 *E. coli* cells. The cells were grown to a density of 0.4 (OD₆₀₀), then induced with 0.5 mM IPTG. Cells were then allowed to grow for an additional 4 hours, at which point they were pelleted and frozen.

Confirmation of the identity of the cloned cDNA as encoding the authentic RB47 protein was accomplished by examining protein expressed from the cDNA by immunoblot analysis and by RNA binding activity assay. The recombinant RB47 protein produced when the RB47 cDNA was expressed was recognized by antisera raised against the *C. reinhardtii* RB47 protein. The *E. coli* expressed protein migrated at 80 kDa on SDS-PAGE, but the protein was actually 69 kDa, as determined by mass spectrometry of the *E. coli* expressed protein. This mass agrees with the mass predicted from the cDNA sequence. A 60 kDa product was also produced in *E. coli,* and recognized by the antisera against the *C. reinhardtii* protein, which is most likely a degradation or early termination product of the RB47 cDNA. The recombinant RB47 protein expressed from the RB47 cDNA is recognized by the antisera raised against the *C. reinhardtii* protein at levels similar to the recognition of the authentic *C. reinhardtii* RB47 protein, demonstrating that the cloned cDNA produces a protein product that is immunologically related to the naturally produced RB47 protein. In order to generate a recombinant equivalent of the endogenous native RB47, the location of the 47 kDa polypeptide was mapped on the full-length recombinant protein by comparing mass spectrometric data of tryptic digests of the *C. reinhardtii* 47 kDa protein and the full-length recombinant protein. Thus, peptide mapping by mass spectrometry has shown that the endogenous RB47 protein corresponds primarily to the RNA binding domains contained within the N-terminal region of the predicted precursor protein, suggesting that a cleavage event is necessary to produce the mature 47 kDa protein. Thus, full-length recombinant RB47 is 69 kDa and contains a carboxy domain that is cleaved *in vivo* to generate the endogenous mature form of RB47 that is 47 kDa.

To determine if the heterologously expressed RB47 protein was capable of binding the *psb*A RNA, the *E. coli* expressed protein was purified by heparin agarose chromatography. The recombinant RB47 protein expressed in *E. coli* was purified using a protocol similar to that used previously for purification of RB47 from *C. reinhardtii.* Approximately 5 g of *E. coli* cells grown as described above were resuspended in low salt extraction buffer (10 mM Tris [pH 7.5], 10 mM NaCl, 10 mM MgCl₂, 5 mM B-mercaptoethanol) and disrupted by sonication. The soluble cell extract was applied to a 5 mL Econo-Pac heparin cartridge (Bio-Rad) which was washed prior to elution of the RB47 protein (Danon and Mayfield, Embo J., 10:3993-4001 (1991)).

The *E. coli* expressed protein that bound to the heparin agarose matrix was eluted from the column at the same salt concentration as used to elute the authentic *C. reinhardtii* RB47 protein. This protein fraction was used in *in vitro* binding assays with the *psb*A 5' UTR. Both the 69 and 60 kDa *E. coli* expressed proteins crosslinked to the radiolabeled *psb*A 5' UTR at levels similar to crosslinking of the endogenous RB47 protein, when the RNA/protein complex is subjected to UV irradiation.

Heparin agarose purified proteins, both from the *E. coli* expressed RB47 cDNA and from *C. reinhardtii* cells, were used in an RNA gel mobility shift assay to determine the relative affinity and specificity of these proteins for the 5' UTR of the *psb*A mRNA. The *E. coli* expressed proteins bound to the *psb*A 5' UTR in vitro with properties that are similar to those of the endogenous RB47 protein purified from *C. reinhardtii.* RNA binding to both the *E. coli* expressed and the endogenous RB47 protein was competed using either 200 fold excess of unlabeled *psb*A RNA or 200 fold excess of poly(A) RNA. RNA binding to either of these proteins was poorly competed using 200 fold excess of total RNA or 200 fold excess of the 5' UTR of the *psb*D or *psb*C RNAs. Different forms of the RB47 protein (47 kDa endogenous protein vs. the 69 kDa *E. coli* expressed protein) may account for the slight differences in mobility observed when comparing the binding profiles of purified *C. reinhardtii* protein to heterologously expressed RB47.

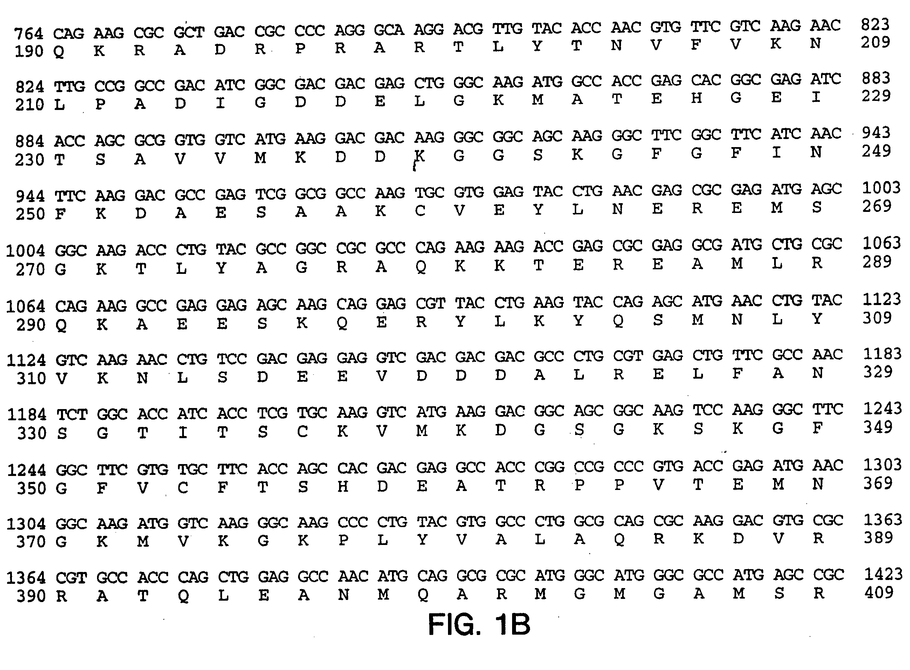

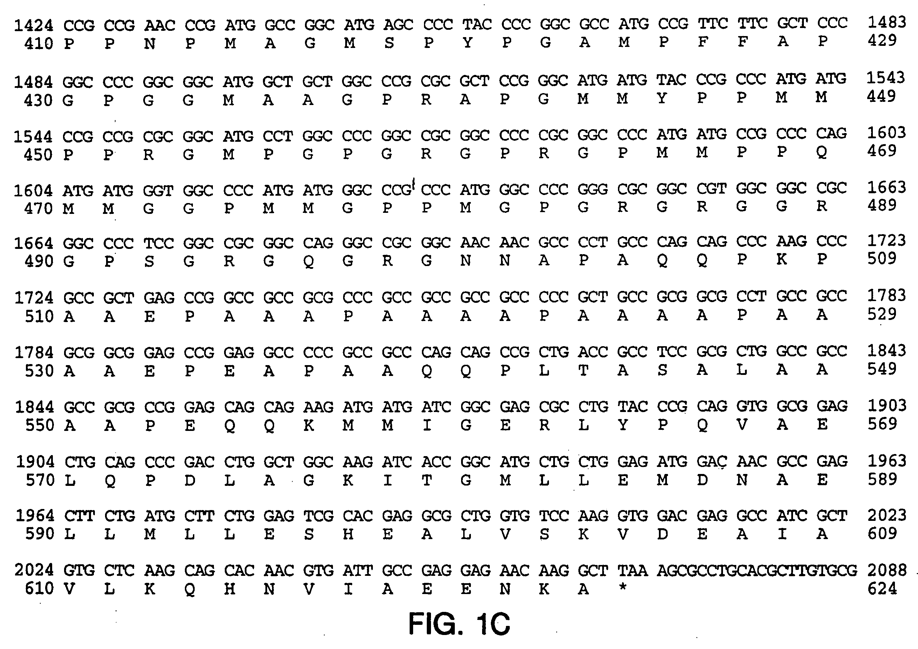

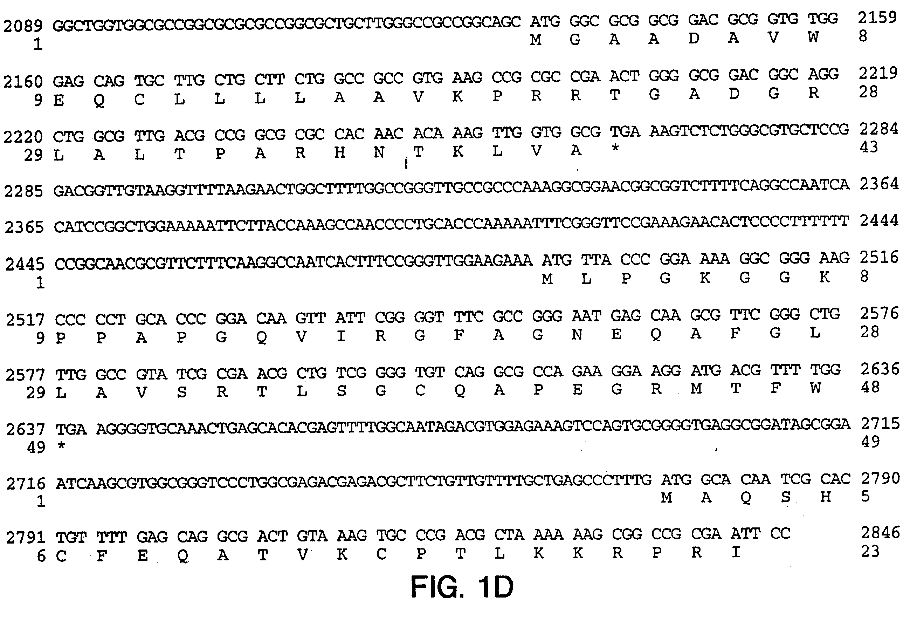

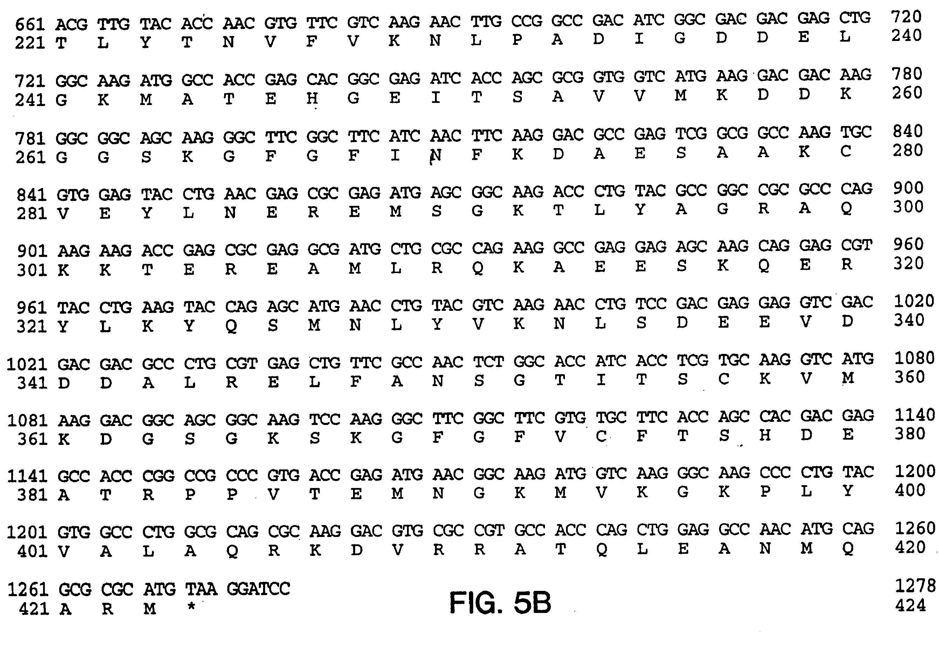

The mature form of RB47 is also produced in recombinant form by the insertion by PCR of an artificial stop codon in the RB47 cDNA at nucleotide positions 1403-1405 with a stop codon resulting in a mature RB47 recombinant protein having 402 amino acids as shown in Figure 1. An example of this is shown in Figure 5 for the production of a recombinant histidine-modified RB47 mature protein as described below. The complete RB47 cDNA is inserted into an expression vector, such as pET3A as described above, for expression of the mature 47 kDa form of the RB47 protein. In the absence of the inserted stop codon, the transcript reads through to nucleotide position 2066-2068 at the TAA stop codon to produce the precursor RB47 having the above-described molecular weight characteristics and 623 amino acid residues.

Recombinant RB47 is also expressed and purified in plant cells. For this aspect, *C. reinhardtii* strains were grown in complete media (Tris-acetate-phosphate [TAP] (Harris, The Chlamydonas Sourcebook, San Diego, CA, Academic Press (1989)) to a density of 5x10⁶ cells/mL under constant light. Cells were harvested by centrifugation at 4°C for 5 minutes at 4,000 g. Cells were either used immediately or frozen in liquid N₂ for storage at -70°C.

Recombinant RB47 protein was also produced as a modified RB47 protein with a histidine tag at the amino-terminus according to well known expression methods using pET19-D vectors available from Novagen, Inc., Madison, WI. The nucleotide and amino acid sequence of a recombinant histidine-modified RB47 of the mature 47 kDa form is shown in Figure 5 with the nucleotide and amino acid sequence also listed in SEQ ID NO 14. Thus the nucleotide sequence of a histidine-modified RB47 is 1269 bases in length. The precursor form of the RB47 protein is similarly obtained in the expression system, both of which are modified by the presence of a histidine tag that allows for purification by metal affinity chromatography.

The recombinant histidine-modified RB47 purified

The recombinant histidine-modified RB47 purified through addition of a poly-histidine tag followed by Ni⁺² column chromatography showed similar binding characteristics as that described for recombinant precursor RB47 described above.

### C. Constructs Containing RB60 Nucleotide Sequence

In one approach to obtain purified recombinant RB60 protein, the full-length RB60 cDNA prepared above was cloned into the *E*. *coli* expression vector pET3A (Studier et al., Methods Enzymol., 185:60-89 (1990)), also commercially available by Novagen, Inc., Madison, WI and transformed into BL21 *E. coli* cells. The cells were grown to a density of 0.4 (OD₆₀₀), then induced with 0.5 mM IPTG. Cells were then allowed to grow for an additional 4 hours, at which point they were pelleted and frozen.

Recombinant histidine-modified RB60 was also expressed with a pET19-D vector as described above for RB47 that was similarly modified. Purification of the recombinant RB60 proteins was performed as described for RB47 thereby producing recombinant RB60 proteins for use in the present invention.

The RB60 coding sequence is also mutagenized for directional ligation into an selected vector for expression in alternative systems, such as mammalian expression systems.

### D. Constructs Containing an RB47-Encoding Sequence and an RB60-Encoding Sequence

To prepare an expression cassette for encoding both RB47 and RB60, one approach is to digest plasmid pD1/RB47/RB60 prepared above with XhoI and XbaI to isolate the fragment for both encoding sequences. The fragment is then inserted into a similarly digested expression vector if available or is further mutagenized to prepare appropriate restriction sites.

Alternatively, the nucleotide sequences of RB47 and RB60, as described in Example 2, are separately prepared for directional ligation into a selected vector.

An additional embodiment of the present invention is to prepare an expression cassette containing the RB47 binding site along with the coding sequences for RB47 and RB60, the plasmid pD1/RB47/RB60 prepared above is digested with NdeI and XhoI to prepare an expression cassette in which any desired coding sequence having similarly restriction sites is directionally ligated. Expression vectors containing both the RB47 and RB60 encoding sequences in which the RB47 binding site sequence is utilized with a different promoter are also prepared as described in Example 4A.

### E. Constructs Containing an RB47 Binding Site Nucleotide Sequence, Insertion Sites for a Desired Heterologous Coding Sequence, and an RB47-Encoding Sequence

In another permutation, a plasmid or expression cassette is constructed containing an RB47 binding site directly upstream of an inserted coding region for a heterologous protein of interest, and the RB47 coding region. The final construct described herein for use in a prokaryotic expression system makes a single mRNA from which both proteins are translated.

The plasmid referred to as pD1/RB47 is prepared as described above in Example 4A. A diagram of this plasmid with the restriction sites is shown in Figure 6.

Expression of pD1/RB47 in E. *coli* to produce recombinant RB47 and the recombinant heterologous protein is performed as described in above. The heterologous protein is then purified as further described.

To produce an expression cassette that allows for insertion of an alternative desired coding sequence, the plasmid pD1/RB47 is digested with NdeI and XhoI resulting in a vector having restriction endonuclease sites for insertion of a desired coding sequence operably linked to a RB47 binding site and RB47 coding sequence on one transcriptional unit.

### F. Constructs Containing an RB47 Binding Site Nucleotide Sequence. Insertion Sites for a Desired Heterologous Coding Sequence, and an RB47-Encoding Sequence

In another permutation, a plasmid or expression cassette is constructed containing an RB47 binding site directly upstream of an inserted coding region for a heterologous protein of interest, and the RB60 coding region. The final construct described herein for use in a prokaryotic expression system makes a single mRNA from which both proteins are translated. In this embodiment, a separate construct encoding recombinant RB47 as described in Example 4B is co-transformed into the *E. coli* host cell for expression.

The plasmid referred to as pD1/RB60 is prepared as described above for pD1/RB47 in Example 4A with the exception that XhoI and XbaI sites are created on RB60 rather than RB47.

Expression of pD1/RB60 in E. *coli* to produce recombinant RB60 and the recombinant heterologous protein is performed as described in above with the combined expression of RB47 from a separate expression cassette. The heterologous protein is then purified as further described.

To produce an expression cassette that allows for insertion of an alternative desired coding sequence, the plasmid pD1/RB60 is digested with NdeI and XhoI resulting in a vector having restriction endonuclease sites for insertion of a desired coding sequence operably linked to a RB47 binding site and RB60 coding sequence on one transcriptional unit.

### G. Constructs Containing RB47 Binding Site Nucleotide Sequence and Heterologous Coding Sequences

### 1) Expression of Recombinant Tetanus Toxin Single Chain Antibody

The examples herein describe constructs that are variations of those described above. The constructs described below contain an RB47 binding site sequence and a heterologous coding sequence. The activating protein RB47 was endogenously provided in the chloroplast and or plant cell. In other aspects however as taught by the methods of the present invention, the chloroplast is further transformed with an RB47-expression construct as described above for overexpression of RB47 to enhance translation capacities.

A strain of the green algae *Chlamydomonas reinhardtii* was designed to allow expression of a single chain antibody gene in the chloroplast. The transgenically expressed antibody was produced from a chimeric gene containing the promoter and 5' untranslated region (UTR) of the chloroplast *psb*A gene prepared as described above, followed by the coding region of a single chain antibody (encoding a tetanus toxin binding antibody), and then the 3' UTR of the *psb*A gene also prepared as described above to provide for transcription termination and RNA processing signals. This construct is essentially pD1/Nde including a heterologous coding sequence having a 3' XbaI restriction site for ligation with the 3' *psb*A gene and is diagramed in Figure 7.

The *psb*A-single chain construct was first transformed into *C. reinhardtii* chloroplast and transformants were then screened for single chain gene integration. Transformation of chloroplast was performed via bolistic delivery as described in US Patents 5,545,818 and 5,553,878, the disclosures of which are hereby incorporated by reference. Transformation is accomplished by homologous recombination via the 5' and 3' UTR of the *psb*A mRNA.

As shown in Figure 8, two of the transformants that contained the single chain chimeric gene produced single chain antibodies at approximately 1% of total protein levels. The transgenic antibodies were of the correct size and were completely soluble, as would be expected of a correctly folded protein. Few degradation products were detectable by this Western analysis, suggesting that the proteins were fairly stable within the chloroplast. To identify if the produced antibody retained the binding capacity for tetanus toxin, ELISA assays were performed using a mouse-produced Fab, from the original tetanus toxin antibody, as the control. The chloroplast single chain antibody bound tetanus toxin at levels similar to Fab, indicating that the single chain antibody produced in *C. reinhardtii* is a fully functional antibody. These results clearly demonstrate the ability of the chloroplast to synthesis and accumulate function antibody molecules resulting from the translational activation of an RB47 binding site in an expression cassette by endogenous RB47 protein in the chloroplast.

### 2) Expression of Bacterial Luciferase Enzyme Having Two Subunits

For the production of molecules that contain more than one subunit, such as dIgA and bacterial luciferase enzyme, several proteins must be produced in stoichiometric quantities within the chloroplast. Chloroplast have an advantage for this type of production over cytoplasmic protein synthesis in that translation of multiple proteins can originate from a single mRNA. For example, a dicistronic mRNA having 5' and 3' NdeI and XbaI restriction sites and containing both the A and B chains of the bacterial luciferase enzyme was inserted downstream of the *psb*A promoter and 5' UTR of the pD1/Nde construct prepared in Example 4A above. In this construct, the bacterial LuxAB coding region was ligated between the *psb*A 5' UTR and the *psb*A 3' end in an *E. coli* plasmid that was then transformed into *Chlamydomonas reinhardtii* cells as described above for expression in the chloroplast. A schematic of the construct is shown in Figure 9. Single transformant colonies were then isolated. A plate containing a single isolate was grown for 10 days on complete media and a drop of the luciferase substrate n-Decyl Aldehyde was placed on the plate and the luciferase visualized by video-photography in a dark chamber. Both proteins were synthesized from this single mRNA and luciferase activity accumulated within the chloroplast as shown in Figure 10. Some mRNA within plastids contained as many as 5 separate proteins encoded on a single mRNA.

### 3) Expression of Dimeric IgA

To generate dimeric IgA, the construct shown in Figure 11 is engineered so that the *psb*A promoter and 5' UTR are used to drive the synthesis of the light chain and heavy chains of an antibody, and the J chain normally associated with IgA molecules. The nucleic acid sequences for the dimeric IgA are inserted into the RB47 binding site construct prepared in Example 4A. The construct is then transformed into *C. reinhardtii* cells as previously described for expression of the recombinant dIgA.

Production of these three proteins within the plastid allows for the self assembly of a dimeric IgA (dIgA). Production of this complex is monitored in several ways. First, Southern analysis of transgenic algae is used to identify strains containing the polycistronic chimeric dIgA gene. Strains positive for integration of the dIgA gene are screened by Northern analysis to ensure that the chimeric mRNA is accumulating. Western blot analysis using denaturing gels is used to monitor the accumulation of the individual light, heavy and J chain proteins, and native gels Western blot analysis will be used to monitor the accumulation of the assembled dIgA molecule.

By using a single polycistronic mRNA in the context of RB47 regulated translation, two of the potential pitfalls in the assembly of multimeric dIgA molecule are overcome. First, this construct ensures approximately stoichiometric synthesis of the subunits, as ribosomes reading through the first protein are likely to continue to read through the second and third proteins as well. Second, all of the subunits are synthesized in close physical proximity to each other, which increases the probability of the proteins self assembling into a multimeric molecule. Following the production of a strain producing dIgA molecules, the production of dIgA on an intermediate scale by growing algae in 300 liter fermentors is then performed. Larger production scales are then performed thereafter.

The foregoing specification, including the specific embodiments and examples, is intended to be illustrative of the present invention and is not to be taken as limiting. Numerous other variations and modifications can be effected without departing from the true spirit and scope of the invention.

## Claims

1. An expression cassette for the expression of a desired eukaryotic molecule within a plastid comprising a suitable promoter operably linked to a eukaryotic transgene of interest.

2. The expression cassette of claim 1, wherein said eukaryotic molecule comprises an antibody.

3. The expression cassette of claim 1 or 2, wherein the promoter is a homologous promoter.

4. The expression cassette of claim 1 or 2, wherein the promoter is a *psbA* promoter.

5. The expression cassette of any one of claims 1 to 4, further comprising a 5' UTR.

6. The expression cassette of claim 5, wherein the 5' UTR further comprises a RB47 binding site sequence.

7. The expression cassette of claim 5, further comprising a 3' UTR.

8. The expression cassette of any one of claims 1 to 7, wherein the plastid comprises a chloroplast.

9. The expression cassette of claim 2, wherein the antibody is a single chain antibody.

10. The expression cassette of claim 2, wherein the antibody is a dimeric antibody.

11. The expression cassette of any one of claims 1 to 8, wherein the desired molecule comprises a luciferase enzyme.

12. The expression cassette of any one of claims 1 to 3 and 5 to 11, wherein the promoter is constitutive.

13. The expression cassette of any one of claims 1 to 3 and 5 to 11, wherein the promoter is inducible.

14. The expression cassette of any one of claims 1 to 3 and 5 to 13, wherein the promoter is a eukaryotic promoter.

15. The expression cassette of claim 1 or 2, wherein the promoter is a prokaryotic promoter.

16. The expression cassette of any one of claims 1 to 15, further comprising an origin of replication.

17. The expression cassette of any one of claims 1 to 16, further comprising a selectable marker.

18. A cell comprising the expression cassette of any one of claims 1 to 17.

19. The cell of claim 18, wherein the cell is a plant cell.

20. The cell of claim 19, wherein the plant cell comprises a plastid.

21. The cell of claim 20, wherein the plastid comprises a chloroplast.

22. The cell of claim 19, wherein the plant cell comprises a mitochondria.

23. The cell of claim 18, wherein the cell is an algae cell.

24. The cell of claim 23, wherein the cell is a *Chlamydomonas reinhardtii* cell.

25. A method for producing a eukaryotic protein of interest comprising transforming a plastid with the expression cassette of any one of claims 1 to 17, allowing the cell to grow, and harvesting the protein.

26. The method of claim 25, wherein the plastid is comprised within a plant cell.

27. The method of claim 25, wherein the plastid is comprised within an algae cell.

28. The method of claim 25, wherein the transformation occurs *in vitro.*

29. The method of claim 25, wherein the transformation occurs *in vivo.*

30. The method of claim 25, wherein the transformation occurs *ex vivo.*

31. The expression cassette of claim 1, wherein the promoter is a heterologous promoter.

32. The expression cassette of claim 1, wherein the promoter is a bacterial promoter, bacteriophage promoter, T3 promoter or a T7 promoter.

33. The expression cassette of claim 31 or 32, wherein the promoter is a constitutive promoter or an inducible promoter.

34. A DNA construct for expression of a transgene within a plastid comprising a promoter functionable in a plastid operably linked to said transgene.

35. The DNA construct of claim 34, wherein said transgene comprises an antibody.

36. The expression cassette of any one of claims 31 to 35, further comprising a 5' UTR.

37. The expression cassette of any one of claims 31 to 36, wherein the plastid comprises a chloroplast.

38. The expression cassette of any one of claims 31 to 37, further comprising an origin of replication.

39. The expression cassette of any one of claims 31 to 38, further comprising a selectable marker.

40. A construct comprising the following components in the 5' to 3' direction of transcription:
a promoter functional in a plant plastid;
a ribosome binding site joined to said promoter;
a DNA sequence encoding at least one eukaryotic peptide; and
a transcription termination site;
wherein said DNA sequence encoding said at least one eukaryotic peptide is heterologous to said plant plastid.

41. The construct according to claim 40, wherein said ribosome binding site is from a plastid leader sequence.

42. The construct according to claim 40 or 41, wherein said DNA sequence encodes a plant nuclear peptide.

43. A plant cell plastid containing the construct according to any one of claims 40 to 42.

44. A cell of a plant or seed containing the plant plastid according to claim 43.

45. A construct comprising the following components in the 5' to 3' direction of transcription:
a promoter functional in a plant plastid;
a ribosome binding site joined to said promoter, said ribosome binding site heterologous to said promoter;
a DNA sequence encoding at least one eukaryotic peptide; and
a transcription termination site.

46. The construct according to claim 45, wherein said DNA sequence encoding said at least one eukaryotic peptide is heterologous to said plant plastid.

47. The construct according to claim 45 or 46, wherein said ribosome binding site is from a plastid leader sequence.

48. The construct according to claim 45 wherein said DNA sequence encodes a plant nuclear peptide.

49. A plant cell plastid containing the construct according to claim 45.

50. A cell of a plant or seed containing the plant plastid according to claim 49.

51. An expression cassette for the expression of a desired eukaryotic molecule within a plastid comprising, in a 5' to 3' direction, a promoter functional in said plastid, said promoter operably linked to a ribosome binding site, a eukaryotic transgene of interest, and a transcription termination site.

52. The construct according to claim 51, wherein said ribosome binding site is from a plastid leader sequence.

53. The construct according to claim 51 or 52 wherein said DNA sequence encodes a plant nuclear peptide.

54. A plant cell plastid containing the construct according to any one of claims 51 to 53.

55. A cell of a plant or seed containing the plant plastid according to claim 54.

56. An expression cassette comprising a promoter functional in a plant plastid, said promoter operably linked to a ribosome binding site, a eukaryotic transgene of interest, and a transcription termination site.

57. The construct according to claim 56, wherein said ribosome binding site is from a plastid leader sequence.

58. The construct according to claim 56 or 57 wherein said DNA sequence encodes a plant nuclear peptide.

59. A plant cell plastid containing the construct according to any one of claims 56 to 58.

60. A cell of a plant or seed containing the plant plastid according to claim 59.

61. A method for producing a protein in a plant cell, wherein said method comprises:
transforming plastids of said plant cell with a construct comprising the following as operably joined components in the 5' to 3' direction of transcription:
a promoter functional in a plant plastid;
a ribosome binding site joined to said promoter, said ribosome binding site heterologous to said promoter;
a DNA sequence encoding a peptide of an eukaryotic cell; and
a transcription termination region; and
growing said plant cells comprising said transformed plastids under conditions wherein said DNA sequence is expressed to produce said eukaryotic peptide in said plastid.

62. The method according to claim 61, wherein said DNA sequence encoding said at least one eukaryotic peptide is heterologous to said plant plastid.

63. The method according to claim 61 or 62, wherein said ribosome binding site is from a plastid leader sequence.

64. A plant cell having a transformed plastid produced according to the method of claim 61.

65. Use of the plant cell according to claim 64 to generate a plant, plant seed or plant part.

66. The method according to any one of claims 61 to 63 wherein said eukaryotic peptide encoded by said DNA sequence from said construct is folded with the correct number of disulfide bonds.

67. The method according to any one of claims 61 to 63 and 66 wherein said eukaryotic peptide encoded by said DNA sequence from said construct in said transformed plant plastid is bioactive when isolated from said transformed plant plastid.

68. A method for producing a protein in a plant cell, wherein said method comprises:
transforming plastids of said plant cell with a construct comprising the following as operably joined components in the 5' to 3' direction of transcription:
a promoter functional in a plant plastid;
a ribosome binding site joined to said promoter;
a DNA sequence encoding at least one eukaryotic peptide; and
a transcription termination region; and
growing said plant cells comprising said transformed plastids under conditions wherein said DNA sequence is expressed to produce said at least one eukaryotic peptide in said plastid.

69. The method according to claim 68, wherein said DNA sequence encoding said at least one eukaryotic peptide is heterologous to said plant plastid.

70. The method according to claim 68 or 69, wherein said ribosome binding site is from a plastid leader sequence.

71. A plant cell having a transformed plastid produced according to the method of any one of claims 68 to 70.

72. Use of the plant cell according to claim 68 to generate a plant, plant seed or plant part.

73. The method according to any one of claims 68 to 70 wherein said at least one eukaryotic peptide encoded by said DNA sequence from said construct is folded with the correct number of disulfide bonds.

74. The method according to any one of claims 68 to 70 and 73 wherein said at least one eukaryotic peptide encoded by said DNA sequence from said construct in said transformed plant plastid is bioactive when isolated from said transformed plant plastid.

75. A method for producing a protein in a plant cell, wherein said method comprises:
transforming plastids of said plant cell with an expression cassette comprising a promoter functional in a plant plastid, a ribosome binding site, a eukaryotic transgene of interest, and a transcription termination site; and
growing said plant cells comprising said transformed plastids under conditions wherein said DNA sequence is expressed to produce said eukaryotic transgene of interest in said plastid.

76. The method according to claim 75, wherein said ribosome binding site is from a plastid leader sequence.

77. A plant cell having a transformed plastid produced according to the method of claim 75 or 76.

78. Use of the plant cell according to claim 77 to generate a plant, plant seed or plant part.

79. The method according to claim 75 or 76 wherein said at least one eukaryotic peptide encoded by said DNA sequence from said construct is folded with the correct number of disulfide bonds.

80. The method according to claim 75, 76 or 79 wherein said at least one eukaryotic peptide encoded by said DNA sequence from said construct in said transformed plant plastid is bioactive when isolated from said transformed plant plastid.

81. A construct comprising the following components in the 5' to 3' direction of transcription:
a promoter functional in a plant plastid;
a 5'-untranslated region encoding a Shine-Dalgarno sequence joined to said promoter;
a DNA sequence encoding at least one peptide derived from an eukaryotic organism; and
a transcription termination region;
wherein said DNA sequence encoding said at least one eukaryotic peptide is heterologous to a plant plastid.

82. The construct according to claim 81, wherein said 5'-untranslated region encoding said Shine-Dalgarno sequence is from a plastid leader sequence.

83. The construct according to claim 81 or 82 wherein said DNA sequence encodes a plant nuclear peptide.

84. A plant cell plastid containing the construct according to any one of claims 81 to 83.

85. A plant cell containing the plant plastid according to claim 84.

86. A construct comprising the following components in the 5' to 3' direction of transcription:
a promoter functional in a plant plastid;
a 5'-untranslated region encoding a Shine-Dalgarno sequence joined to said promoter, said 5'-untranslated region encoding a Shine-Dalgarno sequence heterologous to said promoter;
a DNA sequence encoding at least one peptide derived from an eukaryotic organism; and
a transcription termination region.

87. The construct according to claim 86, wherein said DNA sequence encoding said at least one eukaryotic peptide is heterologous to the plant plastid.

88. The construct according to claim 86 or 87, wherein said 5'-untranslated region encoding said Shine-Dalgarno sequence is from a plastid leader sequence.

89. The construct according to claim 86, 87 or 88 wherein said DNA sequence encodes a plant nuclear peptide.

90. A plant cell plastid containing the construct according to any one of claims 86 to 89.

91. A plant cell containing the plant plastid according to claim 90.

92. A construct comprising the following components in the 5' to 3' direction of transcription:
a promoter functional in a plant plastid;
a Shine-Dalgarno sequence joined to said promoter;
a DNA sequence encoding at least one eukaryotic peptide; and
a transcription termination site;
wherein said DNA sequence encoding said at least one eukaryotic peptide is heterologous to said plant plastid.

93. The construct according to claim 92, wherein said Shine-Dalgarno sequence is from a plastid leader sequence.

94. The construct according to claim 92 or 93 wherein said DNA sequence encodes a plant nuclear peptide.

95. A plant cell plastid containing the construct according to claim 92, 93 or 94.

96. A plant cell containing the plant plastid according to claim 95.

97. An expression cassette for the expression of a desired eukaryotic molecule within a plastid comprising, in a 5' to 3' direction, a promoter functional in said plastid, said promoter operably linked to a 5'-untranslated region encoding a Shine-Dalgarno sequence, a eukaryotic transgene of interest, and a transcription termination site.

98. The construct according to claim 97, wherein said 5'-untranslated region encoding said Shine-Dalgarno sequence is from a plastid leader sequence.

99. The construct according to claim 97 or 98 wherein said DNA sequence encodes a plant nuclear peptide.

100. A plant cell plastid containing the construct according to claim 97, 98 or 99.

101. A plant cell containing the plant plastid according to claim 100.

102. An expression cassette comprising a promoter functional in a plant plastid, said promoter operably linked to a 5'-untranslated region encoding a Shine-Dalgarno sequence, a eukaryotic transgene of interest, and a transcription termination site.

103. The construct according to claim 102, wherein said 5'-untranslated region encoding said Shine-Dalgarno sequence is from a plastid leader sequence.

104. The construct according to claim 102 or 103 wherein said DNA sequence encodes a plant nuclear peptide.

105. A plant cell plastid containing the construct according to claim 102,103 or 104.

106. A plant cell containing the plant plastid according to claim 105.

107. A method for producing a protein in a plant cell, wherein said method comprises:
transforming plastids of said plant cell with a construct comprising the following as operably joined components in the 5' to 3' direction of transcription:
a promoter functional in a plant plastid;
a 5'-untranslated region encoding a Shine-Dalgarno sequence joined to said promoter, said 5'-untranslated region encoding said Shine-Dalgarno sequence heterologous to said promoter;
a DNA sequence encoding at least one peptide of an eukaryotic cell other than a peptide of a plant plastid; and
a transcription termination region;
wherein said DNA sequence encoding said at least one eukaryotic peptide is heterologous to said plant plastid; and
growing said plant cells comprising said transformed plastids under conditions wherein said DNA sequence is expressed to produce said at least one eukaryotic peptide in said plastid.

108. The method according to claim 107, wherein said 5'-untranslated region encoding said Shine-Dalgarno sequence is from a plastid leader sequence.

109. A plant cell having a transformed plastid produced according to the method of claim 107 or 108.

110. Use of the plant cell according to claim 109 to generate a plant, plant seed or plant part.

111. The method according to claim 107 or 108 wherein said eukaryotic peptide encoded by said DNA sequence from said construct is folded with the correct number of disulfide bonds.

112. The method according to claim 107, 108 or 111 wherein said eukaryotic peptide encoded by said DNA sequence from said construct in said transformed plant plastid is bioactive when isolated from said transformed plant plastid.

113. A method for producing a protein in a plant cell, wherein said method comprises:
transforming plastids of said plant cell with a construct comprising the following as operably joined components in the 5' to 3' direction of transcription:
a promoter functional in a plant plastid;
a 5'-untranslated region encoding a Shine-Dalgarno sequence joined to said promoter;
a DNA sequence encoding at least one peptide of an eukaryotic cell other than a peptide of a plant plastid; and
a transcription termination region;
wherein said DNA sequence encoding said at least one eukaryotic peptide is heterologous to said plant plastid; and
growing said plant cells comprising said transformed plastids under conditions wherein said DNA sequence is expressed to produce said at least one eukaryotic peptide in said plastid.

114. The method according to claim 113, wherein said 5'-untranslated region encoding said Shine-Dalgarno sequence is from a plastid leader sequence.

115. A plant cell having a transformed plastid produced according to the method of claim 113 or 114.

116. Use of the plant cell according to claim 115 to generate a plant, plant seed or plant part.

117. The method according to claim 113 or 114 wherein said eukaryotic peptide encoded by said DNA sequence from said construct is folded with the correct number of disulfide bonds.

118. The method according to claim 113, 114 or 117 wherein said eukaryotic peptide encoded by said DNA sequence from said construct in said transformed plant plastid is bioactive when isolated from said transformed plant plastid.

119. A method for producing a protein in a plant cell, wherein said method comprises:
transforming plastids of said plant cell with a construct comprising the following as operably joined components in the 5' to 3' direction of transcription:
a promoter functional in a plant plastid;
a 5'-untranslated region encoding a Shine-Dalgarno sequence joined to said promoter, said 5'-untranslated region encoding said Shine-Dalgarno sequence heterologous to said promoter;
a DNA sequence encoding a peptide of an eukaryotic cell; and
a transcription termination region; and
growing said plant cells comprising said transformed plastids under conditions wherein said DNA sequence is expressed to produce said eukaryotic peptide in said plastid.

120. The method according to claim 119, wherein said 5'-untranslated region encoding said Shine-Dalgarno sequence is from a plastid leader sequence.

121. A plant cell having a transformed plastid produced according to the method of claim 119 or 120.

122. Use of the plant cell according to claim 121 to generate a plant, plant seed or plant part.

123. The method according to claim 119 or 120 wherein said eukaryotic peptide encoded by said DNA sequence from said construct is folded with the correct number of disulfide bonds.

124. The method according to claim 119, 120 or 123 wherein said eukaryotic peptide encoded by said DNA sequence from said construct in said transformed plant plastid is bioactive when isolated from said transformed plant plastid.

125. A method for producing a protein in a plant cell, wherein said method comprises:
transforming plastids of said plant cell with a construct comprising the following as operably joined components in the 5' to 3' direction of transcription:
a promoter functional in a plant plastid;
a 5'-untranslated region encoding a Shine-Dalgarno sequence joined to said promoter;
a DNA sequence encoding at least one eukaryotic peptide; and
a transcription termination region; and
growing said plant cells comprising said transformed plastids under conditions wherein said DNA sequence is expressed to produce said at least one. eukaryotic peptide in said plastid.

126. The method according to claim 125, wherein said 5'-untranslated region encoding said Shine-Dalgarno sequence is from a plastid leader sequence.

127. A plant cell having a transformed plastid produced according to the method of claim 125 or 126.

128. Use of the plant cell according to claim 127 to generate a plant, plant seed or plant part.

129. The method according to claim 125 or 126 wherein said at least one eukaryotic peptide encoded by said DNA sequence from said construct is folded with the correct number of disulfide bonds.

130. The method according to claim 125, 126 or 129 wherein said at least one eukaryotic peptide encoded by said DNA sequence from said construct in said transformed plant plastid is bioactive when isolated from said transformed plant plastid.

131. A method for producing a protein in a plant cell, wherein said method comprises:
transforming plastids of said plant cell with an expression cassette comprising a promoter functional in said plant plastid, a 5'-untranslated region encoding a Shine-Dalgarno sequence, a eukaryotic transgene of interest, and a transcription termination site; and
growing said plant cells comprising said transformed plastids under conditions wherein said DNA sequence is expressed to produce said eukaryotic transgene of interest in said plastid.

132. The method according to claim 131, wherein said 5'-untranslated region encoding said Shine-Dalgarno sequence is from a plastid leader sequence.

133. A plant cell having a transformed plastid produced according to the method of claim 131 or 132.

134. Use of the plant cell according to claim 133 to generate a plant, plant seed or plant part.

135. The method according to claim 131 or 132 wherein said at least one eukaryotic peptide encoded by said DNA sequence from said construct is folded with the correct number of disulfide bonds.

136. The method according to claim 131, 132 or 135 wherein said at least one eukaryotic peptide encoded by said DNA sequence from said construct in said transformed plant plastid is bioactive when isolated from said transformed plant plastid.
